(19) Europäisches Patentamt / European Patent Office / Office européen des brevets

(11) **EP 1 958 532 A2**

(12) **EUROPEAN PATENT APPLICATION**

(43) Date of publication:
**20.08.2008 Bulletin 2008/34**

(51) Int Cl.:
**A45D 19/02** (2006.01)     **A61K 8/00** (2006.01)
**A45D 19/00** (2006.01)

(21) Application number: **07115334.0**

(22) Date of filing: **30.08.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **06.12.2006 US 634648**
**06.10.2006 EP 06021164**
**12.10.2006 EP 06122213**

(71) Applicant: **The Procter and Gamble Company Cincinnati, Ohio 45202 (US)**

(72) Inventors:
• **Glenn Jr., Robert Wayne**
**Liberty Township**
**Ohio, 45044 (US)**
• **Smith, Paul James**
**Twickenham, Middlesex TW2 6EB (GB)**

(74) Representative: **Kohol, Sonia**
**Procter & Gamble Technical Centres Limited**
**Patent Department**
**Rusham Park,**
**Whitehall Lane**
**Egham, Surrey TW20 9NW (GB)**

(54) **Hair treatment application system**

(57) Disclosed herein is a hair treatment application system (10) and methods to use the same. The hair treatment application system (10) comprises the combination of at least one absorbent substrate (30) having specific median pore radius of from about 300 microns to about 3,000 microns and of one or more hair treatment composition (15), each having a viscosity of from about 3,00 cPs to about 150,000 cPs. The hair treatment application system (10) according to the invention allows for easy, non-messy and precise application of hair treatment compositions (15) in particular of highlighting compositions.

Fig.1.

EP 1 958 532 A2

**Description**

FIELD OF THE INVENTION

The present invention relates to application devices which allow for quick, easy and non messy targeted application of cosmetic compositions to keratinous fibres, preferably the present invention is intended for the application of hair treatment compositions to hair.

BACKGROUND OF THE INVENTION

Self applied hair treatment compositions whether they are conditioning or styling compositions, sunscreen protective compositions, high and low lighting, colouring, bleaching, perming or so called special 'party' effects compositions are very desirable for the consumer. Among these hair treatment applications, the specific strand targeting of hair treatment compositions for example such as highlighting or colour- streaks is perceived by consumers as an ideal entry route into the hair colouring arena as it provides an opportunity to alter only a portion of their hair. Thus, it is considered as a low risk and allows the consumers the opportunity to familiarize themselves with the techniques and optimize the resulting effect. Moreover, highlighting is also considered by consumers to provide the opportunity to produce various looks from subtle multi-tonal effects and natural colour variations to more bold and daring effects.

However, a number of technical problems are associated with using self-applied hair treatment compositions without assistance.

A first problem concerns the fact that certain applications, such as applications of dyeing, highlighting and perming compositions, are particularly difficult to be executed satisfactorily without assistance. These applications, in view of the irreversible effects they produce, must occur carefully and precisely.

Another technical problem is associated with the portion of hair where these hair treatments are executed. Highlighting and perming for instance are likely to be carried out on a single hair strand and thus must be performed in an accurate and exact way as the inappropriate application might modify the overall appearance of the look, particularly when the selected hair strands are those close to the user's face. In addition for applications at the back of the head, the user has to act and take decisions on which strands to treat based on a mirror image, easily losing the overall head perspective.

Another technical problem to be considered is that application of hair treatment composition may lead to some mess when those hair treatment compositions are self-applied by the consumer.

This drawback is further amplified, when the hair treatment compositions are unpleasant for consumer contact. This is the case for example with bleaching, colouring and perming compositions as those compositions can easily cause staining or bleaching if dripped onto home surfaces or the consumer's skin. Thus, the consumers whilst having to apply the hair treatment compositions accurately to the hair, have to simultaneously avoid dripping of the hair treatment composition.

In order to facilitate the application of these self-applied hair treatment compositions onto the hair, the manufacturers of such products typically provide the consumer with an applicator. Nevertheless, even when the hair treatment compositions are applied through an applicator, the applicator is often not easy to hold or use and requires considerable practice, patience and experience in order to achieve at least a satisfactory result.

These applicators range from cap and hook type devices, brushes and comb like devices, which are either supplied separately or are designed to be attached to the hair treatment composition bottle. None of these applicators is particularly satisfactory. The cap and hook method requires the consumer to place an apertured cap over the hair and then to select hair strands which are to be subjected to the hair treatment through the apertures with a hook. The brushes, wand and comb devices require the consumer to load the device with the treatment composition and then apply along the length of a selected hair strand, reloading as required. The comb devices typically have a chamber to load a predetermined quantity of composition. The comb and bottle devices require simultaneous squeezing of the bottle to release the treatment composition whilst combing the hair. These applicators require the consumer to personally determine the width of the hair strand to be treated and to separate it from the rest of the hair. Alternatively, only a predetermined strand width selection can be treated and strand separation is not required, thus precluding any variation. Finally, it is highly undesirable that any contamination of the untreated remaining hair with the self-applied hair treatment composition occurs.

Thus, these applicators do not provide the consumer with the easy and quick hair treatment application that is desired. Particularly problematic are the difficulties to apply product to the root area, either to cover newly grown grey hair or to adjust the colour or bleach the virgin hair. The overall characteristic of the hair at the root line is different and due to its close vicinity to the scalp, it is really difficult to handle. Exact placement at the root line is difficult to achieve and contamination of neighbouring hair areas is a common problem.

Furthermore the applicators also do not allow for single root to tip application of the composition unless on very short hair, requiring the consumer to stop, to reload and then to start the application again by reapplying at mid length.

Whilst some consumers may be reasonably satisfied with the end result achieved with the currently available products, due to the time and effort required many consumers still migrate to the services offered by professional hair salon stylists in order to attain the desired result.

The use of hair salon stylists allows the consumer to achieve personal customization of the end look. The salon professionals have a number of applicators and techniques at their disposal which together with training and years of experience allow for the provision of very varied results. However, even within the hair salon environment, the specific application on hair strands of a treatment composition is also a lengthy process. In order to accurately apply the composition and minimize messiness, the consumer is required to regularly spend a number of hours at the salon in order to complete the process. Because of the long time and effort employed by the stylists to achieve the expected end results a very high revenue is demanded for their services. This is not available for all consumers' budgets and thus, home application highlighting products are also highly desirable in terms of cost in comparison with the professional hair stylists.

There is hence still a need to provide a hair treatment application system which can easily, quickly and precisely, deliver at home as well as at professional salons, hair treatment compositions particularly hair highlighting compositions.

Some attempts to address these problems are known in the art. Application articles composed of multiple layers of different materials have been described in US 2002/142027 and US 2005/0079192. US 2002/142027 discloses an article, which comprises a cavity to receive part of the human body. The cavity has a cover defined as to have a composite structure of at least two layers, one of which must be permeable to a thin solvent such water. An adhesive matrix is situated between those two layers and comprises the active substance to be applied. US 2005/0079192 relates to an article impregnated with a cosmetic composition. The article is composed of an absorbent structure and a contact structure. The contact structure comes into contact with a moist surface and has the characteristics of being on one side permeable to the water present on the moist surface and of being hydrophobic on the side adjacent to the absorbent structure.

Sponges able to deliver cosmetic compositions are disclosed in DE 10259016. Such sponges have absorbance capacity and retention capacity so as to deliver all the absorbed composition by applying only minimal pressures.

Diverse are the attempts proposed by the prior art to solve the technical problem of precisely delivering a hair treatment composition on a defined strand with applicator devices avoiding dripping of the hair treatment composition and/or independently on the orientation of the applicator device thus allowing self-application.

US 6626599 discloses an applicator comprising a cylinder-shaped reservoir containing a hair treatment composition, wherein said reservoir has a lateral opening where an elastic deformable material is lodged. Through that opening the hair strand is set into communication with the composition contained in the reservoir.

JP 1991178630 discloses a hair cosmetic applicator characterized by two hinged plates having attached two bags comprising similar or different compositions. Each bag contains a hole from which the composition is released by opening-closing the hinged plates.

Thus, it remains the need for an applicator device capable of accommodating cosmetic compositions, particularly hair treatment compositions, which allows for their easy application on different receiving surfaces without uncontrolled dripping. In particular, the applicator device should allow the application of hair treatment compositions on hair, particularly on single hair strands or on hair at the root line and should also allow easy application on portions of the head difficult to achieve such as the back of the head.

It has now been surprisingly found that a hair treatment application system, as defined herein after, can significantly improve the highlighting results through a simple, intuitive and non-messy application.

SUMMARY OF THE INVENTION

According to the invention, a hair treatment application system (10) is provided, comprising at least one absorbent substrate (30) and one or more hair treatment compositions (15), wherein said at least one absorbent substrate (30) has a median pore radius of from 300 microns to 3,000 microns and wherein said one or more hair treatment compositions each has a viscosity of from 3,000 cPs to 150,000 cPs. Said viscosity is measured before said one or more hair treatment compositions (15) are applied to said at least one absorbent substrate (30).

Said at least one absorbent substrate (30) of said hair treatment applicator system (10) has preferably an absorption capacity for said one or more hair treatment composition (15) of from 10 to 80 grams of liquid per gram of absorbent substrate.

The present invention further relates to methods of treating the hair with said hair treatment application system (10) and kit comprising said hair treatment application system (10).

BRIEF DESCRIPTION OF THE DRAWINGS

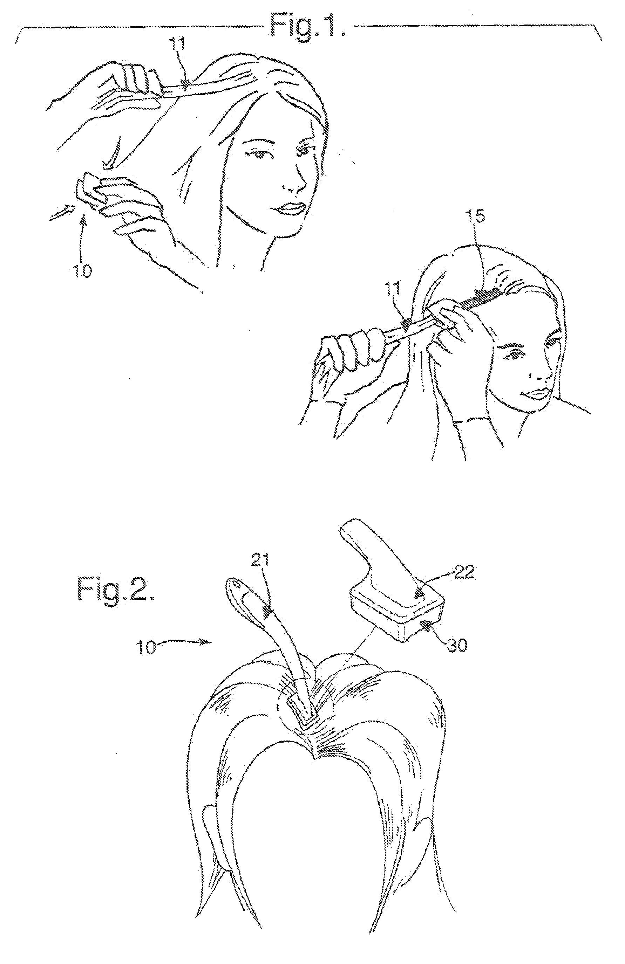

Fig. 1 shows said hair treatment application system (10) and the method of use thereof. On the left side of Fig. 1, the hair treatment application system (10) is held with one hand, then as shown in the right side it is contacted with the hair strand (11) and swiped from the root-line to the tips to apply the hair treatment composition (15).

Fig. 2 shows an embodiment of the hair treatment applicator system (10) according to the invention comprising an absorbent substrate (30) attached to a plate (22), wherein said plate (22) is connected to a handle (21) for ease of use.

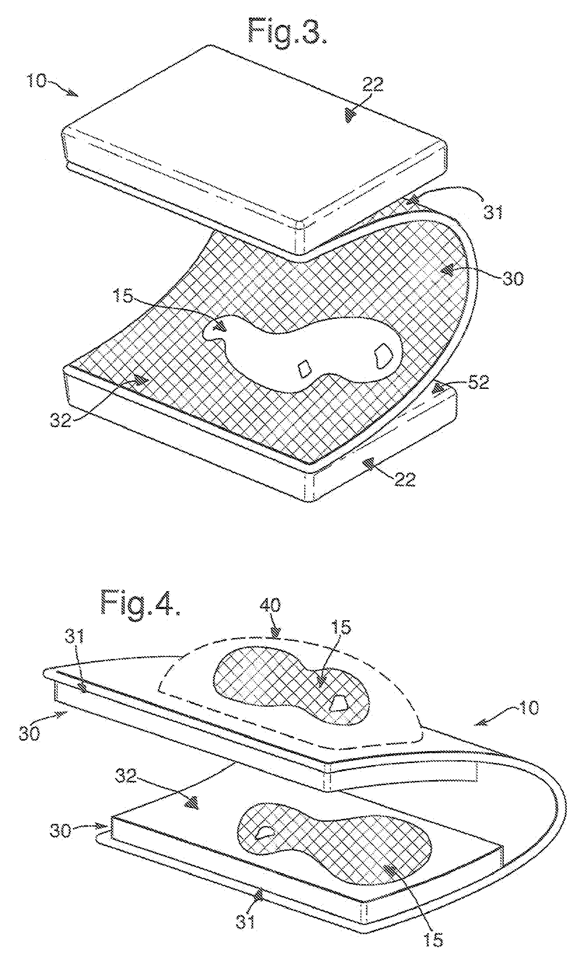

Fig. 3 shows a hair treatment application system (10) according to the invention comprising a hair treatment composition (15) applied onto the inner surface (32) of an absorbent substrate (30). The absorbent substrate (30) is attached on its outer surface (31) to the internal surfaces (52) of two plates (22). The plates are thus connected via said absorbent substrate (30).

Fig. 4 shows a hair treatment application system (10) according to the invention comprising two absorbent substrates (30) attached via their outer surfaces (31) to a flexible pliable support comprising a container (40). A hair treatment composition (15) is applied onto one of the two absorbent substrates' (30) inner surface (32) and into the container (40).

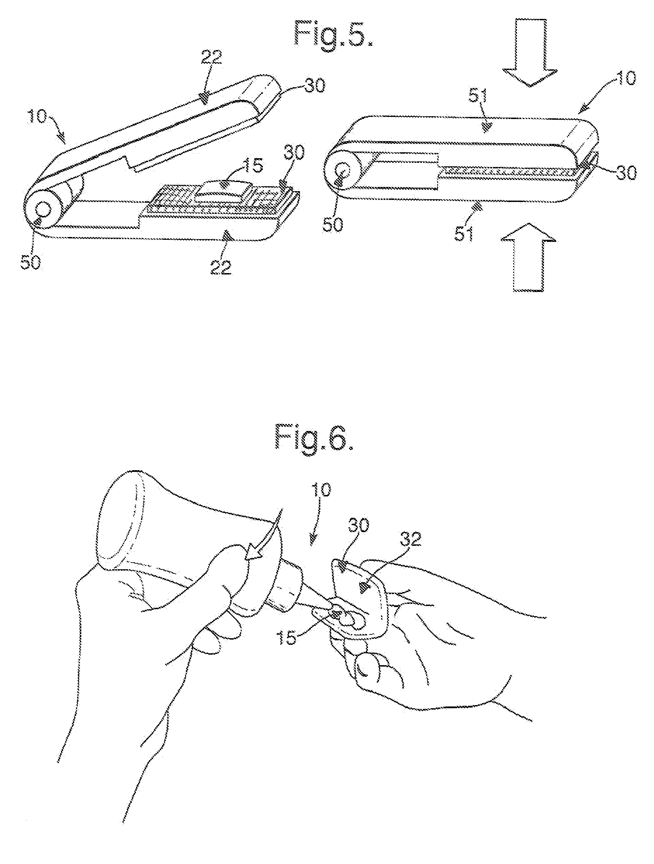

Fig. 5 shows a hair treatment application system (10) comprising two absorbent substrates (30) attached to two plates (22) connected via a hinge (50), wherein one absorbent substrate (30) comprises a hair treatment composition (15) (left). By applying a force onto the external surfaces (51) of the hinged plates (22) the absorbent substrates (30) come into contact.

Fig. 6 shows the loading of a hair treatment composition (15) on the inner surface (32) of an absorbent substrate (30) of a hair treatment application system (10).

## DETAILED DESCRIPTION OF THE INVENTION

For the purpose of this invention, the term hair refers to both living hair i.e. on a living body and to non-living hair i.e. in a wig, hairpiece or other aggregation of non-living keratinous fibre. Mammalian, preferably human hair is intended. However, wool, fur and other keratinous fibre may be suitable to be used with the hair treatment application system (10) according to the invention.

The term hair strand, for the purpose of this invention, refers to at least two keratinous fibres, especially hair, in particular human hair and it should be construed as hair bundle.

As used herein, the term "applied" when referring to a hair treatment composition is to encompass coated, loaded, absorbed, adsorbed and adhered.

While the specification concludes with claims, which particularly point out and distinctly claim the invention, it is believed the present invention will be better understood from the following description.

In examining how to solve the technical problem discussed hereinabove, the present inventors have determined that the delivery of thin fluids from conventional absorbent structures to flat surfaces, i.e., baby wipes, bathroom wipes, kitchen wipes, floor wipes, kitchen cleaning sponges or facial wipes are not applicable for application to hair. Unlike these applications where the thin fluid can be deposited and then smeared across a predominantly flat surface and thereby contained, for delivery to hair, in particular to hair strands, which are cylindrical in shape and without gravimetric confinement to a flat surface, the use of a low viscosity fluid conventional absorbent structures leads to significant consumer undesired dripping and messiness.

The present inventor has now surprisingly identified that the specific selection of the viscosity of the hair treatment compositions of from about 3,000 cPs to about 150,000 cPs in combination with the specific selection of the median pore radius of absorbent substrates of from about 300 microns to about 3,000 microns are critical in order to deliver the desired technical effect.

If the viscosity of the hair treatment composition is too high, it is not easily loaded onto the absorbent substrate and the delivery from the absorbent substrate is too difficult, moreover inter-mixing of two or more differing hair treatment compositions, which is desirable for reactive hair treatment applications such as oxidative dyeing and bleaching including highlighting, if required, can be prohibited. On the other hand if the viscosity of the hair treatment composition is too low the loading may be inefficient causing messiness as the hair treatment composition is not sufficiently retained within the absorbent substrate, causing undesirable dripping and messiness.

As demonstrated hereinafter by the technical experimental data, the minimum viscosity of the hair treatment compositions, measured according to the test method described hereafter, is of at least 3,000 cPs. Carbopol® 956 solutions (available from Noveon) prepared at various concentrations to afford a comprehensive range of viscosities were tested with diverse types of absorbent substrates having a wide range of median pore radii. The Carbopol® 956 solutions were prepared by adding Carbopol® 956 to deionized water while mixing the solution using an overhead stirrer until all the Carbopol® 956 dissolved. Additional Carbopol® 956 was added in the solution until the required viscosity was achieved. Each of the Carbopol® 956 solutions was loaded into the chosen absorbent substrates. The absorbent substrates were then compressed as described in the minimum viscosity limit test method herein after. Each experiment was repeated three times and the results were evaluated using the Student t-test ($\propto$ = 0.05) and statistically different averages are denoted by different letters in square brackets for each absorbent substarte. The average percentage of the loaded Carbopol® 956 solution that had dripped out from the absorbent substrate under compression and the corresponding viscosities are shown in Table 1.

Table 1: Effect of viscosity changes versus median pore radius of the absorbent substrate.

| Absorbent substrates | BBA Fiberweb Tenotex P101 | | Libeltex 01-766 DI-8 | | Recticel Bulpren S28280 | |
|---|---|---|---|---|---|---|
| Median Pore Radii | 75 microns | | 550 microns | | 1,400 microns | |
| Viscosity [cPs] | Average | Std Dev. | Average | Std Dev. | Average | Std Dev. |
| 1000 | 29.93 [A] | 1.16 | 32.95 [A] | 1.50 | 10.08 [A] | 1.48 |
| 1500 | 26.26 [B] | 3.60 | 30.29 [B] | 1.24 | 7.93 [B] | 1.1.88 |
| 2000 | 12.96 [C] | 2.42 | 17.66 [C] | 0.92 | 3.59 [C] | 1.60 |
| 3000 | 0 [D] | 0 | 8.92 [D] | 0.74 | 0 [D] | 0 |
| 3500 | 0 [D] | 0 | 0 [E] | 0 | 0 [D] | 0 |
| 5000 | 0 [D] | 0 | 0 [E] | 0 | 0 [D] | 0 |
| 7500 | 0 [D] | 0 | 0 [E] | 0 | 0 [D] | 0 |
| 10000 | 0[D] | 0 | 0 [E] | 0 | 0 [D] | 0 |

These results clearly illustrate that as the viscosity increases the percentage of Carbopol® 956 solution that drips out from the absorbent substrate significantly decreases. Surprisingly, it has been found that, independently from the absorbent substrate chosen, dripping was substantially absent when the absorbent substrates were loaded with a Carbopol® 956 solution having viscosity of at least about 3,000 cPs

To further support the above discussed experimental data on the hair treatment composition according to the invention, a consumer testing with 12 panellists was also undertaken. Each panellist was given an absorbent substrate (Libeltex 01-766 DI-8, median pore radius 550 microns) to test and to evaluate its messiness and dripping The absorbent substrate was mounted on one side of a plate as described herein after in the Consumer Panelist study test methods section. Two of so prepared plates were hinged together so as to form a clip with the absorbent substrates facing one the other. One absorbent substrate per clip was loaded with solutions of Carbopol® 956 having a viscosity of 1,000 cPs. Other clips were prepared with Carbopol® 956 solutions having viscosities of 2,500 cPs and 5,000 cPs.

The results were evaluated with the Fisher's LSD statistical test ($\propto$ = 0.05), revealing the average grade for both the Carbopol® 956 solutions at viscosity of 1,000 and 2,500 cPs viscosity fluids are statistical identical for both questions and that the one at 5,000 cPs is significantly different for each question and rated as being less messy. These results, shown in table 2, support the technical experimental data that the viscosity required to avoid messiness is in the range between 2,500 cPs to 5,000 cPs. Statistically different averages are denoted by different letters in square brackets.

Table 2: Consumer testing for messiness.

| Question | Considering the sample you have just used, how would you rate its mess? |
|---|---|
| Grade | Scale: 0 - 8<br>0 = Not Messy at All<br>4 = Somewhat Messy<br>8 = Extremely Messy |
| Viscosity [cPs] | Average of All Grades |
| 1000 | 3.83 [B] |
| 2500 | 3.92 [B] |
| 5000 | 2.00 [A] |

The present inventors have also surprisingly found that a very specific type of absorbent substrate is required. Specifically, the absorbent substrates of the present invention are selected such that they posses a median pore radii that can accommodate one or more hair treatment compositions having a minimum viscosity of at least 3,000 cPs. The porous nature of absorbent substrates is accurately described by the median pore radius. This is conventionally established via the TRI Autoporosimeter® methodology for samples with median pore radii less than or equal to 1000 microns and by optical imaging for samples with median pore radii greater than 1000 microns as defined herein after. The effect of median pore radius of absorbent substrates useful within the scope of the present invention was determined using a gravimetric fluid loading as described herein after in the minimum median pore radius test method section. This method represents at best how a consumer may load absorbent substrates with hair treatment compositions. The absorbent substrates chosen were BBA Fiberweb Tenotex P101 (median pore radius of 75 microns); Freudenberg AL 1060 (300 microns); Libeltex 01-766 DI-8 (550 microns); PGI FB-215 (700 microns) and Recticel Bulpren S28280 (1,400 microns). Each absorbent substrate was loaded with Carbopol® 956 solutions having a viscosity of 3,000 cPs, 5,000 cPs and 10,000 cPs, respectively. Each experiment was repeated three times and results were evaluated using the Student t-test ($\propto$ = 0.05) and statistically different averages are denoted by different letters in square brackets. The average amount (in grams) of Carbopol® 956 solution loaded into each gram of absorbent substrate at the corresponding viscosity is shown in Table 3.

Table 3: Effect of the minimum median pore radius of the absorbent substrate on loading.

| Absorbent substrate | BBA Fiberweb Tenotex P101 | | Freudenberg AL 1060 | | Libeltex 01-766 DI-8 | |
|---|---|---|---|---|---|---|
| Median Pore Radii | 75 microns | | 300 microns | | 550 microns | |
| Viscosity [cPs] | Average | Std Dev. | Average | Std Dev. | Average | Std Dev. |
| 3000 | 1.25 [E] | 0.08 | 6.15 [D] | 0.92 | 30.24[B] | 2.62 |
| 5000 | 0.70 [E] | 0.03 | 5.41 [D] | 0.53 | 24.86 [C] | 0.56 |
| 10000 | 0.40 [E] | 0.04 | 2.82 [D] | 0.30 | 17.50 [C] | 0.61 |
| Absorbent substrate | PGI FB-215 | | Recticel Bulpren S28280 | | | |
| Pore Radii | 700 microns | | 1,400 microns | | | |
| Viscosity [cPs] | Average | Std Dev. | Average | Std Dev. | | |
| 3000 | 37.54 [A] | 0.85 | 24.33 [C] | 0.48 | | |
| 5000 | 33.61 [A] | 1.68 | 27.38 [B] | 2.41 | | |
| 10000 | 22.38 [A] | 0.98 | 21.24 [B] | 0.28 | | |

The results illustrate that by increasing the median pore radius of the absorbent substrate, the amount of Carbopol® 956 solution which can be loaded into each absorbent substrate also increases. Furthermore, the results indicates that Carbopol® 956 solutions having minimum viscosity of 3,000 cPs could not significantly be loaded when the absorbent substrate has a median pore radius of less that 300 microns, such in the case of BBA Fiberweb Tenotex P101 which has a median pore radius of 75 microns.

Thus, for the purpose of the present invention, the median pore radius of the absorbent substrate is of at least about 300 microns. Below this minimum median pore radius, hair treatment compositions of viscosity of at least about 3,000 cPs cannot be accommodated for uses in the intended consumer applications of the present invention.

The maximum median pore radius of the absorbent substrates selected herein was determined to be the maximum radius of an open cell foam that is feasible to manufacture, that is commercially available and that is still capable of holding a shape while it is used for the intended consumer applications of the present invention. The maximum median pore radius open cell foam is provided by Recticel and has a median pore radius of 3,000 microns. Absorbent substrates having median pore radii higher than 3,000 microns have a tendency to lose their mechanical strength and thus are not employed within the scope of the present invention. Thus, the absorbent substrates according to the present invention have a median pore radius of from about 300 microns to about 3,000 microns, preferably from about 400 microns to about 2,500 microns, more preferably from about 450 microns to about 2,000 microns, even more preferably from about 500 microns to about 1,800 microns.

Hair treatment compositions have very diverse viscosities and presently commercially available products have viscosities up to about 600,000 cPs. Within the scope of the present invention, the maximum viscosity of a hair treatment composition that can still be loaded on the absorbent substrates as selected above and delivered to the hair is 150,000 cPs. Two absorbent substrates representative of the above determined median pore radius range, Recticel Pottscorer 410 (about 1,400 microns) and Recticel Bulpren S28280 (about 2,200 micron), were chosen. These two absorbent substrates were then loaded with a Carbopol® 956 solution having viscosity of about 100,000 cPs and three hair bundles were subsequently contacted with the absorbent substrates to coat the Carbopol® 956 solution on the hair. In both cases the absorbent substrates were able to provide an amount in grams of Carbopol® 956 solution per grams of hair that achieved highlights. These results are shown in table 4 below. A similar amount of a commercially available highlighting composition would be sufficient to provide a highlighting effect.

Table 4: Effect of the viscosity on average dosage

| Absorbent substrate | Recticel Bulpren S28280 | | Recticel Pottscorer 410 | |
|---|---|---|---|---|
| Median Pore Radii | 1,400 microns | | 2,200 microns | |
| Mileage | Average | Std Dev. | Average | Std Dev. |
| Highlight 1st | 1.73 | 0.19 | 1.42 | 0.32 |
| Highlight 2nd | 1.50 | 0.30 | 0.68 | 0.30 |
| Highlight 3rd | 1.46 | 0.22 | 0.43 | 0.29 |

Although these results refer to a viscosity value of 100,000 cPs, the one skilled in the art of hair treatment compositions would find it easy to understand that hair treatment compositions having viscosity up to about 150,000 cPs could still be loaded on the absorbent substrates according to the invention and applied to the hair. Thus, the hair treatment compositions according to the present invention are defined to have a viscosity of from about 3,000 cPs to about 150,000 cPs, more preferably from about 5,000 cPs to about 125,000 cPs, more preferably from about 7,000 cPs to about 100,000 cPs, even more preferably from about 9,000 cPs to about 85,000 cPs, wherein said viscosity is measured before application into the absorbent substrates according to the maximum viscosity limit test method described hereinafter.

In addition to the median pore radius, it has also been determined that selecting a defined absorbent capacity may also improve the application of the hair treatment composition onto the hair. The absorbent capacities of the absorbent substrates of the present invention can be described by the maximum uptake of water in grams per gram of substrate (grams per gram). The absorbent capacities of the absorbent substrates of the present invention are preferably in the range of from about 10 to about 80 grams of hair treatment composition per gram of absorbent substrate, preferably from about 15 to about 75 grams per gram, more preferably from about 20 to about 70 grams per gram, and even more preferably from about 25 to about 65 grams per gram of absorbent substrate as determined according to the calculation described herein after in the absorbent capacity test method section.

The inventors have also determined that a selected calliper range of the absorbent substrate may also assist in delivering the required amount of hair treatment composition to the hair. The calliper is measured at the highest point on the absorbent substrate's surface conventionally via a micrometer according to the calliper test method described herein after. The callipers of the absorbent substrates of the present invention are preferably in the range of from about 2 to about 20 mm, preferably from about 3 to about 17 mm, more preferably from about 4 to about 12 mm, and even more preferably from about 5 to about 10 mm.

Similarly, the selection of absorbent substrate materials which have a specific basis weight may also enhance the absorption capacity. Preferably the absorbent substrates of the present invention also have a basis weight of from about 20 to about 300 $g/m^2$, preferably from about 60 to about 250 $g/m^2$, more preferably from about 100 to about 200 $g/m^2$, according to the basis weight test method described herein after.

Without wishing to be bound by theory the present inventors believe that the selected calliper and median pore radius influence the liquid permeability of the absorbent substrate. The liquid permeability is a measure of the ability of a material to convey fluids.

1. ABSORBENT SUBSTRATES

Suitable absorbent substrates for use in the present invention may be selected from non-wovens; wovens; porous foams and foam materials; porous plastics; flexible frits; meshes; and combinations thereof including recycled and composite materials having one or more plies of the same or different materials superimposed physically, joined together continuously (laminated), in a discontinuous pattern, or by bonding the external edges at discrete loci provided that the structures meet the functional requirements described hereinabove.

The absorbent substrates of the present invention are preferably selected from non-wovens and/or porous foams.

Non-woven materials are produced from fibers that may be staple or continuous filaments or be formed in situ and include a manufactured sheet, web or batt or directionally or randomly oriented fibers, bonded by friction, and/or cohesion and/or adhesion. Nonwoven webs and processes for making them may comprise three steps: fiber laying, precursor web formation, and fiber bonding. The fiber laying step may be comprised of the spunlaying, meltblowing, carding, airlaying, wetlaying and combinations thereof, of the fibers comprising the web onto a forming surface. The step of precursor web formation may prevent the fibers comprising the web from coming apart during the bonding step. Precursor web formation may be performed via a pre-bonding step, such as one that is chemical or mechanical in nature. The bonding step may then impart strength to the finished web. The bonding step may be comprised of subjecting the fibers comprising the web to hydroentanglement (HET), cold calendering, hot calendering, air through bonding, chemical bonding, needle punching, and combinations thereof. Suitable non-woven materials may be comprised of natural or synthetic fibers selected from acetate fibers; acrylic fibers; cellulose ester fibers; modacrylic fibers; polyamide fibers; polyester fibers; polyolefin fibers; polyvinyl alcohol fibers; rayon fibers; keratin fibers; cellulose fibers; silk fibers and combinations thereof. The non-wovens may be comprised of mono-component fibers, such as a polyolefin or polyester, or bi-component fibers, such as a sheath/core fiber or side by side fiber of polyethylene/polypropylene or polyethylene/ polyester, or bi-constituent fibers comprised by a blend of two or more thermoplastic polymers.

The preferred non-woven substrates are selected from Carded, Air-laid, and Meltblown non-woven materials or composites. More preferably, the non-woven substrates of the present invention are selected from Carded webs produced by a carding machine with one of more different types of fibres. Even more preferably, the non-woven substrates of the present invention are selected from multi-layer or lofty web which are consolidated by through air bonding or needle-punching, often referred to as batting battings. Examples of suitable Carded non-wovens for use herein include; Libeltex Thermo-contact 01-766 DI-8; Libeltex Loftfill HC2; PGI FB-215; PGI FB-204B, PGI FB-185 and PGI FB-217.

Porous foams and foam materials are made from low density elastomers, plastics, and other materials with various porosities and may be selected from open cellular foams; flexible foams; rigid foams; and reticular foams and syntactic foams which can be fabricated into finished shapes using molding, casting, extrusion, pultrusion, machining, thermal forming, plastic welding, blow molding, rapid prototyping techniques, grinding and/or other specialized processes. The porous foams and foam materials may be composed of a variety of chemical systems including acrylonitrile-butadiene-styrene (ABS); acrylics; epoxy resins; fluoropolymers; isoprene-styrene (SIS) and styrene-butadiene-styrene (SBS); synthetic rubbers or elastomers based on a variety of systems such as silicone, polyurethane and neoprene; nitrile rubbers; plastics or elastomers formed from natural or plant-based raw materials such as natural rubber (polyisoprene) or vulcanized fibre; water-based and water-borne resins and latex materials. Chemical systems for porous foams and foam materials may include ethylene copolymer, expanded polyethylene, polycarbonate, polyester, polyether, polyetherimide, polyimide, polyolefin, polypropylene, polyurethane, phenolic, polyurea, and vinyl.

Porous plastics can be made from wide variety of materials including Polytetrafluoroethylene (PTFE), Polyethylene (PE), Polypropylene (PP), and Polyvinyldifluoride (PVDF). They are created by filling a mold with tiny plastic pellets, subjecting the mold to heat and pressure so the pellets bond where they touch. This part is then heated outside the mold; the part shrinks significantly during this step which strengthens it.

The porous foams are preferably polyurethane foams. Suitable examples of porous foams are available from Recticel International (Belgium) and include Sweepex S 31 CS/R, Bulpren S28280, Bulpren D32133, Filtren T23220, and Filtren TM 23133.

The absorbent substrates (30) of the present invention are provided such that they can easily fit in the hand of a user or an optional application tool described herein after and as shown in Figs. 2 to 5. Any shape of the absorbent substrate (30) may be used such as circular, oblong or rectangular shapes. Each absorbent substrate has an outer (31) and an inner (32) surface. The outer surface (31) faces the user's hand and the inner surface (32) is the surface which is loaded with the hair treatment composition (15) and comes into contact with the hair.

The hair treatment application system (10) of the present invention comprises at least one absorbent substrate (30) as shown in Figs. 1, 2, 3 and 6, preferably at least two absorbent substrates (30) as shown in Figs. 4 and 5.

Finally, the absorbent substrates (30) according to the invention are preferably substantially inert to the hair treatment compositions (15).


2. HAIR TREATMENT COMPOSITION

As discussed hereinabove, the present inventors have surprisingly determined that the viscosity of the hair treatment composition (15) must be selected in combination with the median pore radius of the absorbent substrate (30) in order to provide the expected technical effect. Accordingly it has been surprisingly found that for effective loading and retention of the hair treatment composition (15) into the absorbent substrate (30), said one or more hair treatment compositions (15) each has a viscosity of from about 3,000 cPs to about 150,000 cPs, preferably from about 5,000 cPs to about 125,000 cPs, more preferably from 7,000 cPs to about 100,000 cPs, even more preferably from about 9,000 cPs to about 85,000.

The viscosity of the hair treatment composition (15) is measured before the hair treatment composition (15) is loaded into the absorbent substrate (30). The viscosity is measured for liquid hair treatment compositions (15) only. The term liquid hair treatment composition as used therein means a liquid form of a hair treatment composition such as paste, composition, solutions water-in-oil emulsions or other suitable forms, provided that they are in the form of a liquid when delivered to head and that have viscosity within the range claimed herein. Preferably the hair treatment compositions are applied onto the absorbent substrates (30) in the form of compositions which provide good adhering properties to the absorbent substrate (30). Hydrogels are especially preferred as they provide a source of water that facilitates diffusion and absorption of the hair treatment compositions (15) within the absorbent substrate (30).

According to the present invention, there are a number of ways to provide the desired hair treatment composition mixture in the absorbent substrate. Consequently, the term "before" as used herein has the following meaning for these executions:

When two (or more) identical hair treatment compositions (15) are loaded independently into at least one absorbent substrate (30) and then mixed within said at least one absorbent substrate (30), the viscosity of the hair treatment composition is measured before loading one of said hair treatment composition (15) onto said at least one absorbent substrate (30).

When two different hair treatment compositions (15) are premixed before loading into at least one absorbent substrate (30), the viscosity is measured on the resulting mixed hair treatment composition (15) before the premixed hair treatment composition is loaded into the absorbent substrate.

When two (or more) different hair treatment compositions (15) are independently loaded into at least one absorbent substrate (30) and then mixed within said at least one absorbent substrate (30), the viscosity of each single hair treatment compositions (15) is measured before loading into said at least one absorbent substrate (30). For embodiments involving the loading of two different compositions into the absorbent substrate, which are subsequently mixed within the absorbent substrate, it is preferred that the viscosity of the resulting mixed hair treatment composition has a viscosity of from about 3,000 cPs to about 150,000 cPs. For such embodiments, the viscosity of the resultant mixed hair treatment compositions is determined by mixing the two (or more) hair treatment compositions prior to loading into the absorbent substrate as described hereinafter in the test method section. This specific embodiment is exemplified in examples A and B here after.

When one of the hair treatment compositions (15) is a powder, then the viscosity of the hair treatment composition (15) resulting from the mixing of said powder with another liquid hair treatment composition (15) is measured.

It is also believed that for some hair treatment compositions (15) it is desirable to have a shear thinning rheology to facilitate non-messiness, good performance, and a precise application of the hair treatment composition (15) only within the selected hair strand avoiding cross-contamination to the rest of the hair.

3. HAIR TREATMENT COMPOSITIONS APPLIED TO THE ABSORBENT SUBSTRATES

Any hair treatment composition (15) characterized by having the viscosity within the ranges selected herein can be employed in the underlying invention and can be loaded in the selected absorbent substrates (30) to achieve the technical effect described above. Examples of hair treatment compositions (15) that may be applied to the absorbent substrate(s) (30) are discussed below. Suitable hair treatment compositions (15) include shampoos, conditioners, styling compositions, hair colourants, bleaches, and highlighting compositions.

Accordingly, the compositions may comprise components known, conventionally used, or otherwise effective for use in hair treatment compositions (15) particularly oxidative bleaching and dye compositions which include but are not limited to: developer dye compounds; coupler dye compounds; direct dyes; oxidizing agents; thickeners; chelants; pH modifiers and buffering agents; carbonate ion sources and radical scavenger systems; anionic, cationic, nonionic, amphoteric or zwitterionic surfactants, or mixtures thereof; anionic, cationic, nonionic, amphoteric or zwitterionic polymers, or mixtures thereof; fragrances; dispersing agents; peroxide stabilizing agents; proteins and derivatives thereof, plant materials (e.g. aloe, chamomile and henna extracts); silicones (volatile or non-volatile, modified or non-modified), film-forming agents, ceramides, preserving agents, colour indicators and opacifiers. Some adjuvants referred to above, but not specifically described below, which are suitable are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions) are useful in identifying specific adjuvants to achieve a particular purpose or multipurpose.

A. Solvents

The medium suitable for dyeing may be selected from water or a mixture of water and at least one organic solvent to dissolve the compounds that would not typically be sufficiently soluble in water. Suitable organic solvents for use herein include, but are not limited to: C1 to C4 lower alkanols (e.g., ethanol, propanol, isopropanol), aromatic alcohols (e.g. benzyl alcohol and phenoxyethanol); polyols and polyol ethers (e.g., carbitols, 2-butoxyethanol, propylene glycol, propylene glycol monomethyl ether, diethylene glycol monoethyl ether, monomethyl ether, hexylene glycol, glycerol, ethoxy glycol), and propylene carbonate. Organic solvents are typically present in an amount ranging from about 1% to about 30%, by weight, of the composition. Preferred solvents are water, ethanol, propanol, isopropanol, glycerol, 1,2-propylene glycol, hexylene glycol, ethoxy diglycol, and mixtures thereof.

B. Oxidative Dye Compounds

The compositions of the present invention may include oxidative dye compounds in the form of primary intermediates or couplers. The compounds suitable for use in the inventive compositions (including those optionally added), in so far as they are bases, may be used as free bases or in the form of their physiologically compatible salts with organic or inorganic acids, such as hydrochloric.

Optional couplers are typically present in an amount such that in aggregate the concentration of couplers and the present discrete particle aggregates and/or agglomerates in the composition ranges from about 0.002% to about 10%, preferably from about 0.01% to about 5%, by weight, of the hair dyeing composition. Optional primary intermediates are present in an effective dyeing concentration, typically an amount from about 0.001 % to about 10%, preferably from about 0.01% to about 5%, by weight, of the hair dyeing composition. The total amount of dye compounds in the hair dyeing compositions of this invention will typically range from about 0.002% to about 20%, preferably from about 0.04% to about 10%, more preferably from about 0.1 % to about 7%, by weight, of the hair dyeing composition. These compounds are well known in the art, and include aromatic diamines, aminophenols, aromaticdiols and their derivatives (a representative but not exhaustive list of oxidation dye precursor can be found in Sagarin, "Cosmetic Science and Technology", "Interscience, Special Edn. Vol. 2 pages 308 to 310). It is to be understood that the precursors detailed below are only by way of example and are not intended to limit the compositions and processes herein. These are: toluene-2,5-diamine, p-phenylenediamine, n-phenyl-p-phenylenediamine, resorcinol, 4-chlororesorcinol, m-aminophenol, p-aminophenol, 1-naphthol, 1,5-naphthalenediol, 2,7-naphthalenediol, p-methylaminophenol, hydroxybenzomorpholine, 4-amino-2-hydroxytoluene, 2-methyl-5-hydroxyethylaminophenol, 1,2,4-trihydroxybenzene, phenyl methyl pyrazolone, 2,4-diaminophenoxyethanol, 3-amino-2,4-dichlorphenol, 2-methylresorcinol, n,n-bis(2-hydroxyethyl)-p-phenylenediamine, 2,4,5,6-tetraminopyrimidine, 4-amino-m-cresol, 6-amino-m-cresol, 1,3-bis-(2,4-diaminophenoxy)-propane, hydroxyethyl-p-phenylene diamine, 2-amino-4-hydroxyethylaminoanisole, 5-amino-6-chloro-o-cresol, hydroxyethyl-3,4-methylenedioxyaniline, 2,6-dihydroxy-3,4-dimethylpyridine, 2,6-dimethoxy-3,5-pyridinediamine, dihydroxyindole, 5-amino-4-chloro-o-cresol, hydroxypropyl-bis-(n-hydroxyethyl-p-phenylenediamine), 6-hydroxyindole, isatin, 3-amino-2-methylamino-6-methoxypyridine, 2-amino-3-hydroxypyridine, 2,6-dihydroxyethylaminotoluene, 2,5,6-triamino-4-pyrimidinol, dihydroxyindoline, 1-acetoxy-2-methylnaphthalene, 1-hydroxyyethyl-4,5-diaminopyrazole, 2,2'-methylenebis-4-aminophenol, 2-methyl-1-naphthol, 4-formyl-1-methylquinolinium-p-toluenesulfonate. These can be used in the molecular form or in the form of peroxide-compatible salts.

The hair colouring compositions of the present invention may also include non oxidative hair dyes. i.e. direct dyes which may be used alone or in combination with the above described oxidative dyes. Suitable direct dyes include azo or anthraquinone dyes and nitro derivatives of the benzene series and or melanin precursors and mixtures thereof. Such direct dyes are particularly useful to deliver shade modification or highlights. Particularly preferred are Basic Red 51, Basic Orange 31, Basic Yellow 87 and mixtures thereof.

C. Alkalizing agent

According to the present invention the composition may also comprise at least one source of alkalizing agent. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts, ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia, ammonium carbonate, ammonium carbamate, ammonia, sodium silicate, sodium metasilicate, sodium disilicate, ammonium persulfates, sodium persulfate, potassium persulfate and mixtures thereof.

The compositions of the present invention may comprise from about 0.1 % to about 40% by weight, preferably from about 1.0% to about 35%, most preferably from about 2% to about 30% of an alkalizing agent, preferably ammonium ions.

D. Oxidizing Agent

The compositions may comprise an oxidizing agent, present in an amount sufficient to bleach melanin pigment in hair and/or cause formation of dye chromophores from oxidative dye precursors (including developers and/or couplers when present). Typically, such an amount ranges from about 1% to about 20%, preferably from about 3% to about 15%, more preferably from about 6% to about 12%, by weight, of the developer composition. Inorganic peroxygen materials capable of yielding hydrogen peroxide in an aqueous medium are preferred and include but are not limited to: hydrogen peroxide; inorganic alkali metal peroxides (e.g. sodium periodate and sodium peroxide); organic peroxides (e.g. urea peroxide, melamine peroxide); inorganic perhydrate salt bleaching compounds (e.g. ammonium persulfates, sodium persulfate, potassium persulfate and mixtures thereof). Preferred are hydrogen peroxide and persulphates. The persulfate powders may be mixed with another liquid hair treatment composition as described herein after or alternatively may be immobilized physically via applying the powder particles to the interior and/or exterior surface of the absorbent substrate in such a means that they are physically contained and do not exit the substrate easily under gravity. This can be achieved via a hollow pocket or reservoir within the absorbent substrate that contains the persulfate salt blend.

E. pH Modifiers and Buffering agents

The compositions may further comprise a pH modifier and/or buffering agent in an amount that is sufficiently effective to adjust the pH of the composition to fall within a range from about 3 to about 13, preferably from about 8 to about 12, more preferably from about 8 to about 11. Suitable pH modifiers and/or buffering agents for use herein include, but are not limited to: ammonia, alkanolamides such as monoethanolamine, diethanolamine, triethanolamine, alkali metal and ammonium hydroxides and carbonates, preferably sodium hydroxide and ammonium carbonate, and silicates such as sodium silicate and sodium metasilcate, and ammonium chloride..

F. Carbonate ion source

The compositons of the present invention may further comprise in a preferred embodiment at least one source of peroxymonocarbonate ions, preferably formed insitu from a source of hydrogen peroxide and a carbonate ion source. According to the present invention the compositions thus also may comprise at least a source of carbonate ions or carbamate ions or hydrocarbonate ions or any mixture thereof. Any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate and mixtures thereof. The compositions of the present invention may comprise from about 0.1% to about 15%, preferably from about 0.1% to about 10% by weight, more preferably from about 1% to about 8% by weight of the carbonate ion.

G. Radical scavenger system

The compositions may comprise a radical scavenger, in a sufficient amount to reduce damage to the hair during the coloring process. Typically, such an amount will range from 0.1% to 10%, preferably from 1% to 7%, by weight of the composition. The radical scavenger is preferably selected such that it is not an identical species as the alkalizing agent. The radical scavenger is a species that can react with a carbonate radical to convert the carbonate radical by a series of fast reactions to a less reactive species. Preferred radical scavengers may be selected from the classes of alkanolamines, amino sugars, amino acids and mixtures thereof, and may include, but are not limited to: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, serine, tryptophan and potassium, sodium and ammonium salts of the above and mixtures thereof. Other preferred radical scavenger compounds include benzylamine, glutamic acid, imidazole, di-tert-butylhydroxytoluene, hydroquinone, catechol and mixtures thereof.

H. Chelants

The compositions may comprise chelants in an amount sufficient to reduce the amount of metals available to interact with formulation components, particularly oxidizing agents, more particularly peroxides. Typically such an amount will range from at least about 0.25%, preferably at least about 0.5%, by weight, of the composition. Suitable chelants for use herein include but are not limited to: diamine-N,N'-dipolyacid, monoamine monoamide-N,N'-dipolyacid, and N, N'-bis(2-hydroxybenzyl)ethylenediamine-N,N'-diacetic acid chelants (preferably EDDS (ethylenediaminedisuccinic acid)), carboxylic acids (preferably aminocarboxylic acids), phosphonic acids (preferably aminophosphonic acids) and polyphosphoric acids (in particular straight polyphosphoric acids), their salts and derivatives.

I. Humectants

The compositions may comprise humectants. Typically the amount of humectants in the hair treatment composition will range from at least about 1 to about 50%, preferably from about 5 to about 40%, and more preferably from about 10 to about 30%, by weight, of the composition. Suitable humectants for use herein include but are not limited to: polyhydric alcohols such as glycerin, polyethylene glycol, and propylene glycol, and mixtures thereof.

J. Thickening agents

The hair treatment composition may further comprise a thickening agent. The thickening agent is present at a level of from about 0.01% to about 20%, preferably from about 0.1% to about 10%, more preferably from about 0.3% to about 5%, and even more preferably from about 0.5% to about 3%, by weight of the composition.

Gel network thickener system may be used as thickening agent for the purpose of the present invention. The gel network thickener system comprises at least one low HLB surfactant or amphophile having high melting point and at least one additional second surfactant.

The low HLB surfactant or amphophile has an HLB of 6 or less and a melting point of at least 30°C. Representative examples include following compounds (in the examples below "solid" refers to material state at temperature below 30°C): solid fatty alcohols, solid oxyethylenated fatty alcohols, solid glycol esters, solid oxyethylenated alkyl phenols, solid sorbitan esters, solid sugar esters, solid methyl glucoside esters, solid polyglycerine esters, solid alkyl glyceryl ethers, solid propylene glycol fatty acid esters, cholesterol and ceramides. Preferably the low HLB surfactants are selected from linear or branched fatty alcohols comprising from about 14 to 30 carbon atoms, oxyethylenated fatty alcohols comprising from about 16 to 30 carbon atoms and at most about 2 units of ethylene oxide and glycerol mono esters of fatty acids comprising from about 16 to 30 carbon atoms. Most preferably the low HLB surfactants include cetyl, stearyl, cetostearyl or behenyl alcohols, steareth-2 and glycerol monostearate.

The second surfactant of the gel network thickener system may be anionic, non-ionic or cationic. Examples of anionic surfacnts include, but are not limited to those of the formula RnXmYM, wherein R is a alkyl, alkenyl or alkylaryl group having from 8 to 30 carbon atoms, X is a polar group comprising at least one carbon atom and at least one oxygen or nitrogen atom, Y is an anionic group selected from carboxylates, sulphates, sulphonates or phosphates, n and m are independently 1 or 2 and M is hydrogen or a salt forming cation and mixtures thereof. Examples of non-ionic surfactants include those having an HLB of 7 or more and comprising one or more polyethyleneoxide chains wherein each polyethyleneoxide chain contains on average at least about 50 ethylene oxide units. Also suitable for use as nonionic surfactants are non-ionic surfactants having an HLB of 7 or more which are free of polyethyleneoxide chains. Examples of cationic surfactants include, but are not limited to quaternary ammonium salts or amido-amines having at least one fatty chain comprising from 8 to 30 carbon atoms and mixture thereof. The quaternary ammonium salts have general formula N+ (R1R2R3R4) X-,wherein, R1 is selected from linear and branched radicals comprising about 12 to 30 carbon atoms, R2 is selected from linear and branched radicals comprising about 12 to 30 carbon atoms or the same group as radicals R3 to R4, the radicals R3 to R4, which may be identical or different, are selected from linear and branched aliphatic radicals comprising from about 1 to 4 carbon atoms, and aromatic radicals such as aryl and alkylaryl, the aliphatic radicals may comprise at least one hetero atom such as oxygen, nitrogen, sulphur and halogens, the aliphatic radicals are chosen, for example, from alkyl, alkoxy and alkylamide radicals, and wherein X- is an anion selected from halides such as chloride, bromide and iodide) (C2-C6)alkyl sulphates, such as methyl sulphate, phosphates, alkyl and alkylaryl sulphonates, and anions derived from organic acids, such as acetate and lactate. The cationic surfactant is selected from, for example, a behentrimonium chloride, behenamidopropyltrimonium methosulfate, stearamidopropyltrimonium chloride, arachidtrimonium chloride and mixtures thereof. The amido-amine have general formula R'1 - CONH(CH2)nNR'2R'3: wherein, R'1 is selected from linear and branched radicals comprising about 12 to 30 carbon atoms, the radicals R'2 and R'3, which may be identical or different, are selected from hydrogen, linear and branched aliphatic radicals comprising from about 1 to 4 carbon atoms, and aromatic radicals such as aryl and alkylaryl, the aliphatic radicals may comprise at least one hetero atom such as oxygen, nitrogen, sulphur and halogens, the aliphatic radicals are chosen, for example, from alkyl, alkoxy and alkylamide radicals, and wherein n is integer from 1 to 4. The amido-amine is selected from, for example, behenamidopropyldimethylamine, stearamidopropyldimethylamine and mixtures thereof.

More than one surfactant of the above specified types or any combination of the surfactants can be used and the weight ratio of the low HLB surfactants to the second specified surfactants is from about 100:1 to about 1:10, preferably from 20:1 to 1:2, and more preferably from 10:1 to 1:1.

Amide surfactants are also suitable thickening agents, preferably when mixed with a source of carbonate ions. The amide surfactants may be selected from polyoxyethylene amides or polyhydroxy amides. Polyoxyethylene amides are selected from compounds according to the formula R-(OCH2CH2)x-(OCH2)y-C(O)NH(CH2CH2O)z-H, wherein x is independently selected from 0 to 100, y is 0 or 1, z is independently selected from 1 to 100, and R is independently selected from alkyl, alkenyl or alkylaryl groups having from 8 to 30 carbon atoms or is a polyhydroxy amide according to the formula:

Worm-like micelle phase thickening systems may also be suitable thickening agents for the purpose of the present invention. The worm-like micelle thickening system of the present invention is defined as a thickening system comprising at least one ionic surfactant and an electrolyte source of counter-ions for said ionic surfactant. Suitable ionic surfactants for use herein may be selected from anionic surfactants, cationic surfactants and or mixtures thereof. Suitable thickening agents for use herein also include synthetic polymers such as cellulose derivatives (e.g. methylcellulose, carboxymethylcellulose, hydroxyethylcellulose, hydroxypropylcellulose, hydroxy-propylmethylcellulose, etc.), carbomer polymers (e.g. crosslinked polyacrylic acid copolymer or homopolymer and copolymers of acrylic acid cross linked with a polyalkenyl polyether), hydrophobically modified polyacrylic acid polymers, natural and synthetic gums, karaya gum, guar gum, gelatin, algin, sodium alginate, tragacanth, chitosan, polyethylene oxide, acrylamide polymers, polyacrylic acid, polyvinyl alcohol, polyamines, polyquarternary compounds, ethylene oxide polymers, polyvinylpyrrolidone, cationic polyacrylamide polymers, and mixtures thereof.

Other associative thickening agents include Rohm and Haas (such as Acrysol® ICS-1 and Aculyn® 22 and 28 thickeners, which are hydrophobically modified alkali-soluble acrylic polymer emulsions and Aculyn® 44 and 46 thickener, which is a hydrophobically modified nonionic polyol).

Preferred thickening agents are chosen from polymers (including gelling agents), gel phases referred to as creams or emulsions and combinations thereof.

A representative but not exhaustive list of polymers and thickening agents can be found in "The Encyclopaedia of Polymers and Thickeners for Cosmetics" compiled and edited by Robert Y. Lochhead, PhD and William R. Fron, Department of Polymer Science, University of Southern Mississippi.

Suitable gel phase referred to as creams or emulsions may be selected from cetyl alcohol, stearyl alcohol, fatty acids and mixtures thereof.

4. OPTIONAL APPLICATION TOOL

An application tool (20) can optionally assist the delivery of the hair treatment composition (15) when used in conjunction with the hair treatment application system (10) according to the invention.

Suitable application tools (20) include, but are not limited to, tools composed by a single piece or by a plurality of same or different parts. Examples include, but are not limited to plates attached to handles, tongs, applicators to be fixed to the user's finger, pliable foils, filmic or cellulose based substrates or carrier materials, clips, clamps, shells, pincers, tweezers, scissors, single or multiple moulded parts with flexible elastomers or live hinges, folding combs, not permeable materials, interconnected plates (22), preferably hinged plates (22), as shown in Fig. 5. The plates (22) may present curved depressions on their surfaces and may include permanently connected or removable compartments (40) for the hair treatment composition (15) as shown in Fig. 4.

The application tool (20) provides ease of handling of the absorbent substrate (30) by the user. In addition, the application tool (20) minimizes contact of the user with the hair treatment composition (15) and also improves the application process of the hair treatment composition (15) to the hair. Preferably, the application tool (20) may allow exact selection of the hair strand to be treated and may be independent of orientation. The application tool (20) may provide means of separating one from more hair treatment compositions (15) e.g. oxidising agent and alkalising agent prior to activation and mixing to form a highlighting composition. The application tool (20) may help provide partial or full containment of one or more hair treatment compositions (15) within the hair treatment application system (10). Moreover, the application tool (20) may aid the delivery of the hair treatment composition (15) onto or in close proximity to the absorbent substrate (30). The application tool (20) may further provide a way to store the hair treatment composition (15) over the required product shelf-life.

The optional application tool (20) may also be provided with one or more means suitable to perform the loading of the hair treatment composition (15) into the absorbent substrates (30) or into the application tool (20). Examples of said means are, but not limited to, nozzles, orifices, valves, one-way valves.

The application tool (20) may be manufactured from any known material capable of supporting the absorbent substrate (30) and the hair treatment composition (15). Suitable materials are a polymer resin such as a polyolefin e.g. polypropylene, polyethylene or polyethylene terephthalate. Other polymers could be used including polyvinylchloride, polyamide, acetyl, acrylonitrile butadiene styrene, acrylic, acrylonitrile styrene acrylate, ethylene vinyl alcohol, polycarbonate, polystyrene, silicone or thermo plastic elastomer or copolymers where appropriate or a flexible pliable substrate such as paper, board, metal based substrates and aluminium foil, filmic substrates or multiple laminations or combinations of multiple layers of said materials.

The application tool (20) or at least a portion thereof should preferably fit within the user's hand and it is preferably ergonomically designed to better adapt to the shape of the human hand for ease of use during application when used in either hand.

The application tool (20) comprises surfaces for attachment or association with the absorbent substrate (30). The application tool (20) has at least one surface to which one or more absorbent substrates (30) can be attached. Preferably, the surfaces of the application tool (20) extend beyond the surface of the absorbent substrate (30).

The application tool (20) is removable or permanently attached to the absorbent substrates (30). Any methods suitable to attach the absorbent substrate (30) to the application tool (20) may be employed herein providing that said method does not destroy or alter the performance of the absorbent substrate (30). The absorbent substrate (30) may be attached to the application tool (20) by using heat welding including pressure, ultrasonic forces, radio or high frequencies. The absorbent substrate (30) may also be attached to the application tool (20) through adhesive, including two-side tape, thermo-set, hot melt and cold seal, adhesion or extrusion lamination. Mechanical interlock or entanglement such as Velcro®, clamping, snap locks, sealing beads, locking pins and magnetism may also be used to adhere the absorbent substrate (30) to the application tool (20). The hair treatment composition (15) may be loaded before or after the adhesion of the absorbent substrate (30) to the application tool (20), but for those methods that could alter or inactivate the hair treatment composition (15), the absorbent substrate (30) is loaded after it has been attached to the application tool (20).

The optional application tool (20) may comprise one or more of the same or different hair treatment compositions (15) contained in one or more of its parts. Examples include, but are not limited to, compartments (40) separated by barrier material or liners removable via peeling, rupturing, puncturing, breaking, tearing, piercing, sliding, folding, compression, ball bearing. Said compartments (40) may be fixed, attachable, removable and may be disposed once the hair treatment compositions (15) have been released. Different hair treatment compositions (15) may come into contact through fluid inter-mixing mechanisms either prior to or during usage including, but not limited to, dispensing from two different compartments (40) through a torturous pathway via pressure.

The hair treatment compositions (15) may be dispensed simultaneously or sequentially. The hair treatment compositions (15) may be dispensed in close proximity to the absorbent substrate (30) or into a void space within the application tool (20). In one embodiment shown in Fig. 2, the application tool (20) is formed by a single elongated piece having a handle (21) on one side and a plate (22) on the other side to which the hair treatment application system (10) is adhered.

In another embodiment for use herein the application tool (20) is formed by a single piece obtained by moulding a flexible and pliable support as shown in Fig. 4. In one preferred embodiment the application tool (20) comprises two plates (22), preferably connected via any suitable means, such as the absorbent substrate itself (30) as shown in Fig. 3 or via a hinge (50) as shown in Fig. 5. The hinge (50) can be formed in a number of ways including: a "live" injection moulded hinge, a strap hinge or flexible strip (folding link that is connected between the two components), a kiss-cut or a crease. In another embodiment, when the optional tool comprises two plates, each of said plates comprises an absorbent substrate on one side and a fitting means on the other side to accommodate the user's fingers, being the hand of the user the connection.

The plates (22) may be flat or curved and each plate (22) has an external (51) and internal (52) surface. In the preferred embodiment, an absorbent substrate (30) is attached to the internal surface (52) of each plate (20), via its outer surface (31). The plates (22) may be identical in design for ease of manufacture and assembly, but in other embodiments the plate's design may be different. Both the first and second plates (22) may be manufactured in a number of ways including: injection moulding, bi-injection moulding, thermo or vacuum forming of a blister type shell, lamination onto a carrier plastic or board material in the horizontal plane.

The hair treatment composition (15) can be pre-impregnated in the absorbent substrates (30), pre-loaded either in the absorbent substrate (30) or in one or more compartments (40) of the application tool (20) or the hair treatment compositions (15) can be applied just prior to use in the entirety or just a portion of the absorbent substrates (30) or into the application tool's compartments (40). In either case, the hair treatment compositions (15) may be identical or different. In one embodiment of the present invention, the absorbent substrate (30) may be provided with a release liner to protect the absorbent substrate (30) itself and or the hair treatment composition (15) from contamination when not in use. The release liner may be resealed after use to the absorbent substrate (30), or to the application tool (20), if present. The release liner may be aluminium or plastic (low density polyethylene/aluminium laminate/polyethylene terephthalate) peel-able foils and may be made of a gas resistant material, especially for hair treatment composition (15) comprising hydrogen peroxide. When the absorbent substrate (30) are attached to an optional application tool (20) said absorbent substrates (30) can be protected by external contamination by closure or covering means applied or mounted on the application tool (20) itself such as screwable caps, which are removed during use but that may be easily reapplied to protect the hair treatment composition (15) and the absorbent substrates (30) after use. Such closure or covering means serve also to avoid contamination of the user's home with the hair treatment composition (15) comprised therein when the application tool (20) is laid down during use.

5. HAIR TREAMENT COMPOSITION LOADING AND MIXING

According to the present invention said one or more hair treatment compositions (15) may be loaded into at least one absorbent substrate (30). In embodiments where only one absorbent substrate (30) is present, one or more hair treatment compositions (15) may be applied either on the same or, preferably, on different sections of the absorbent substrate (30). In embodiments where more than one substrate is present, said absorbent substrates (30) may be the same or different and one or more hair treatment compositions (15) may be applied onto the same or different sections of each absorbent substrate (30).

When the optional application tool (20) is present the hair treatment composition (15) may be loaded into the application tool (20), preferably into an application tool's separate compartment (40) or within a void space of the application tool (20).

The hair treatment composition (15) can be loaded into the absorbent substrate (30) or into the application tool (20) by any means. In one embodiment the hair treatment composition (15) is loaded into the absorbent substrate (30) directly by applying the hair treatment composition (15) for example from a bottle as shown in Fig. 6.

In another embodiment, upon the application of the hair treatment composition (15) into at least one absorbent substrate (30), the absorbent substrate (30) is subjected to mechanical pressure, preferably said pressure applied by the fingers of the user as shown in Fig. 1 (left), to allow the hair treatment composition (15) to evenly distribute within the whole absorbent substrate (30) by flowing through its pores.

The hair treatment compositions (15) may be loaded by pressure or by vacuum. When vacuum is used, the air within the absorbent substrate (30) is displaced by the hair treatment composition (15). In the case of pressure, the hair treatment composition (15) is moved into the absorbent substrate (30) or into a compartment of the application tool (20) by means of an air pump, hand-bellows or alike. The same or different hair treatment compositions (15) can be loaded in different sections of the absorbent substrates (30) or in different compartments (40) of the application tool (20). The hair treatment compositions (15) may be loaded in different time, such as one hair treatment composition (15) is loaded and only when that hair treatment composition (15) is absorbed evenly within the absorbent substrate (30), then a second and any further hair treatment compositions (15) are applied.

Any of the above described method of loading and applying the hair treatment composition (15) can be either performed manually or by mechanically operated machines, especially when the loading is executed during manufacture of the hair treatment application system (10). One or more methods may be employed either simultaneously or subsequently one to another. In another embodiment, the hair treatment composition (15) may be loaded into a vacuum formed or blister tray which is sealed by a higher barrier laminate material.

The amount of hair treatment composition (15) applied on the absorbent substrates (30) will depend upon the size and capacity of the absorbent substrate (30), type of hair treatment composition (15) and the desired end results.

6. METHOD OF USE

The present invention also relates to a method to treat the hair by contacting the hair with the hair treatment application system (10). By contacting the hair with the hair treatment application system (10) the hair treatment composition (15) is delivered to the hair. The user may hold the hair treatment application system (10) on the absorbent substrate's outer surface (31) as shown in Fig. 6. The inner surface (32) of the absorbent substrate (30) is the surface that is brought into contact to the hair. The user contacts the hair with the absorbent substrate's (30) inner surface (32) as shown in Fig. 1 (right). To perform this action the user may simply contact the absorbent substrate (30) to the hair, preferably at the root-line and coat the hair treatment composition (15) as shown in Fig. 2. Otherwise the absorbent substrate (30) may be folded along one of its dimensions as shown in Fig. 1 so that the inner surface (32) can be wrapped around a hair strand (11) while the user holds in only one hand the absorbent substrate's (30) outer surfaces (31), preferably the user holds the absorbent substrate (30) between the thumb and the index finger. The selected hair strand (11) is held with the other hand while the absorbent substrate (30) is positioned onto the hair strand (30), preferably at the root line. The hair treatment composition (15) is then applied by swiping the hair treatment applicator system (10) along the entire length of the selected hair strand (30) as shown in Fig. 1.

In the embodiments of the hair treatment application system (10) as shown in Figs. 4 and 5, comprising two absorbent substrates (30), the user selects the hair strand (11) and places it between the two absorbent substrates (30), preferably at the root line. The absorbent substrates (30) are clamped around the hair strand (11) and then moved along the length of the hair to the tips in order to apply the hair treatment composition (15). In one embodiment of the hair treatment application system (10) according to the invention, a first and a second absorbent substrates (30) are present. The first absorbent substrate (30) comprises a first hair treatment composition (15) and a second absorbent substrate (30) comprises a second hair treatment composition (15). The first and second hair treatment composition (15) may be the same or different. Preferably, when the first and second hair treatment compositions (15) are different, the first hair treatment composition (15) is capable of reacting with the second hair treatment composition (15) to form a third hair treatment composition (15). When the first and second compositions (30) are brought into contact, the hair treating composition (15) may be formed immediately or a further activation step may be required. For example the reaction may be heat-, water- or pressure-activated. In the case of water-activation, water may be applied to the hair prior to being placed between the absorbent substrates (30). In another embodiment the absorbent substrate (30) itself may be contacted with water prior to being applied on the strands of hair. Combination of the activation steps described above is also foreseeable. The mixing of said first and second hair treatment composition (15) is achieved by compressing and squeezing once, preferably more than once, said first and second absorbent substrates (30) before the application of the hair treatment composition (15) occurs.

In another embodiment two absorbent substrates (30) as defined herein are attached to two hinged plates (22) and a hair strand (11) is then selected and located between the two absorbent substrates (30). Application of the hair treatment composition (15) loaded into the absorbent substrates (30) or mixing of the hair treatment compositions (15) loaded into the absorbent substrates (30) is obtained by applying a force on the external surface (51) of both plates (22) so that they swing by a certain degree with respect to their hinge (50) as shown in Fig. 5. This mixing, by pressing the two plates (22) together, compresses and squeezes the attached absorbent substrates (30) and forces the hair treatment compositions (15) contained therein to mix. This mixing method can be performed prior or during application to hair as many times as needed to evenly mix the hair treatment compositions before to proceed with the application onto the hair strand (11). In this embodiment, the absorbent substrate (30) that is attached to the first plate (22) is designated as an area to which a hydrogen peroxide based composition is loaded, whereas the absorbent substrate (30) that is attached on the second plate (22) is designated as an area to which an alkaliser composition is loaded. When the hydrogen peroxide composition and the alkalizer composition are mixed together they form a highlighting composition. It is irrelevant which plate (22) is designated as the first and second plate (22) as long as the hydrogen peroxide composition and the alkalizer composition are loaded in different absorbent substrates (30) or different sections thereof.

In one embodiment, a first hair treatment composition (15) can be incorporated within a single absorbent substrate (30) and is capable of reacting with a second hair treatment composition (15) that is applied to the hair separately to the application of the first hair treatment composition (15), preferably on a separate absorbent substrate (30). The first and the second hair treatment compositions (15) may be contacted together physically either immediately prior or during consumer application to the hair.

In another embodiment a first hair treatment composition (15) is applied to the hair from the absorbent substrate (30) as a pre- or post-treatment to a second hair treatment composition (15) and any further hair treatment composition (15) which can be applied via conventional liquids dispensed from bottles, as a cream, as a mousse, as a gel etc or with an additional hair treatment conventional device or an hair treatment application system (10) of the present invention.

As shown in Figs. 1 and 2, the absorbent substrates (30) of the present invention are not intended to be applied to hair in a stationary manner but rather they are moved against the hair surface with the use of shear forces, i.e., swiping of individual hair strands, rubbing along root-line, rubbing into hair, wiping surface of hair, pulled through hair etc., thereby depositing the hair treatment composition (15) evenly along the entire length of the hair as required.

In embodiments where a liner is present, the user peels off the liner, loads the absorbent substrate (30) and applies the hair treatment composition (15) to the hair. During the application one or more liners can be resealed to protect the absorbent substrates (30) and the hair treatment compositions (15) or to avoid contamination of the user's home furniture with the hair treatment composition (15). In embodiments when the absorbent substrates (30) are preloaded by the manufactures and then sealed with liners, the user removes the liners before application of the hair treatment compositions (15). Once the liners have been removed, the user can either proceed to use or apply additional hair treatment compositions (15) by loading them onto the absorbent substrates (30) or into the optional application tool (20) as described above. One or more liners can be peeled off simultaneously or one individually after the other.

Finally, the application of the hair treatment composition (15) may occur on wet or dry hair and optionally, a rinsing or a shampooing step can be included between application of the first and any further compositions (15) to the hair.

## 7. KITS

The hair treatment application system (10) according to the present invention may be provided as a component of a kit comprising single packaged containers as described herein below. The kit according to the invention may comprise one or more individually packaged compositions comprising shampoo compositions, conditioning compositions, styling compositions, hair colourant compositions, hair bleaching, highlighting compositions or combination thereof. In one embodiment of the present invention, a first container may comprise an oxidative dye precursors and an alkalizing agent whereas a second container may comprise an oxidizing agent. In certain other embodiments of the kit, a first container may comprise an alkalizing agent , preferably a source of ammonium ions and a second container may comprise an oxidizing agent, preferably hydrogen peroxide. Additional containers may be present in the kit, such as individually packaged composition comprising additional components such as oxidising agents, conditioners, chelants, radical scavengers, solvents, direct dyes, shampoo, buffering agents, colouring agents thickeners, enzymes, anionic, non ionic, amphoteric and cationic surfactants, carriers, antioxidants, stabilizers, perfumes, masking fragrances, herb and plant extracts, pearlescent, opacifiers, hair swelling agents and/or polymers, humectants, moisturizers, viscosity enhancers, gelling agents, chelators, UV filters, antimicrobials , preservatives, proteins or mixtures thereof.

The kit according to the present invention may further comprise additional components such as means to select the hair strand, means to load the hair treatment application system (10) according to the present invention, means to mix the hair treatment compositions (15), combs or brushes, gloves, caps with holes, tweezers, tongues, hooks or combination thereof.

In one embodiment the kit according to the invention comprises the hair treatment application system (10) as described herein and gloves. In another embodiment the kit further comprises an individually packaged composition comprising an oxidizing agent and an individually packaged composition comprising an alkalizing agent. Preferably said alkalizing agent comprises a persulfate salt.

In certain embodiments, the hair treatment application system (10) according to the present invention is provided with an optional application tool (20), The application tool (20) may be provided assembled but also unassembled in the kit and instruction how to build the unassembled application tool (20) of the present invention may be further provided in the kit described above. The kit comprising the hair treatment application system (10) according to the present invention may further comprise instructions for consumers indicating how to load and/or use the hair treatment application system (10), said instruction being recorded in any type of media such as the package of the kit itself, paper material, compact disk, DVD or the hair treatment application system (10) itself or the optional application tool (20).

## 8. TEST METHODS

Median Pore Radius

There is no single method to measure median pore radii across the range of absorbent substrate selected for the purpose of the present invention. The median pore radius of the absorbent substrate was measured firstly via TRI Autoporosimeter™. For values obtained above 1000 microns the measurement was repeated via optical imaging and the value obtained with the optical imaging was taken as the final value.

The TRI Autoporosimeter™ is an automated, computer controlled instrument for measuring pore radii and their corresponding cumulative pore volumes (B. Miller and I. Tyomkin, Journal of Colloid and Interface Science, 162 (1994), 163-170). The median pore radius of a sample is the equivalent cylindrical radius where the cumulative volume of fluid absorbed/desorbed equals 50 % of the saturation capacity of the test sample. The equivalent cylindrical radius can be afforded from the Laplace equation (I) that relates the hydrostatic pressure (pressure at 50 % saturation) to pore radius:

$$R = \frac{2\gamma \cos\theta}{\Delta P} \qquad (I)$$

where R is the effective radius, $\gamma$ is the surface tension of the wetting liquid, $\theta$ is the contact advancing or receding contact angle of the liquid and $\Delta P$ is the pressure difference in hydrostatic head pressure across the sample. The median equivalent pore radius was typically determined from the desorption measurements, where $\theta$ is the receding contact angle. If the 50% value does not correspond to a pre-selected equivalent radius, then it can be determined graphically by interpolation.

The test sample, typically 50 mm in diameter, is placed in a measurement cell on a Millipore glass filter membrane (porosity of 1.2 microns) attached to a Monel support plate. The filter membrane and Monel metal support are prepared according to the manufacture's recommendations and attached via an epoxy based paint (Krylon High Gloss available from Swerin-Williams Corp.). Measurements are conducted with an applied pressure (confining weight) of 0.1 psi to ensure the test sample is in contact with the fluid test membrane.

The measurement cell is connected to a reservoir of the test liquid placed upon a balance. The TRI Autoporosimeter™ is used with n-hexadecane to wet the absorbent substrate. The weight (volume) of the liquid absorbed/desorbed by the absorbent substrate as a function of applied air pressure is recorded by the balance. As the air pressure increases and decreases, different size radius groups desorb and absorb liquid, respectively. The radius volume of each group is equal to this volume of liquid transferred. To ensure that all samples are tested under the same close to equilibrium conditions, the following parameters are recommended: (i) Equilibrium constant (liquid flow at each pressure step) is less than 90 mg/min, (ii) Balance weight data is collected every 15 seconds until desired equilibrium constant value is reached. The results are afforded as capillary pressure (cm), as a function of fluid weight on the balance (g).

Test samples with median pore radii greater than 1000 microns were determined by optical imaging. A test sample of about 50 x 50 mm was placed in an Olympus Stereo-Microscope connected to a digital camera. The dark field illumination technique was used to enhance the contrast and improve the visual appearance of the 3D structure. The applied magnifications are chosen to a) obtain regions of interest (ROIs) that represent the structure of the samples and b) enable reliable measurements of the pore sizes. The pore area of all the focused pores were manually measured in the ROIs and subsequently analyzed with the Olympus analySIS FIVE software to afford the accumulative surface pore area as a function of equivalent circular pore radius. The median pore radius of a sample is the equivalent circular radius where the cumulative pore area equals 50% of the total surface pore area. The measurements include pores that are not completely parallel to the focal plane, but observed from a viewing angle (due to the 3D structure).

The median pore radius was reported to the nearest micron ($\mu$m). The median pore radii and the other technical characteristic of some of the absorbent substrates according to the present invention are presented in table 5 below. Table 5 also shows absorbent substrates that do not satisfy the specifications claimed herein (indicated by an asterisk in table 5).

Absorbent Capacity

The absorption capacity was defined for a porous substrate as the mass of liquid absorbed per unit mass of dry solid substrate (see, for instance,. Absorbent Technology, by Chatterjee and Gupta, Elsevier, 2002):

$$ C = \frac{\rho_l}{\rho} \times \frac{\phi}{1-\phi} $$

where $\rho$ is the density of the material making up the substrate, $\rho_1$ is the density of the liquid (for the stated Absorbent Capacities the density of pure water is used - 1.00 g/cm$^3$) and $\phi$ is 1 minus the quotient of $\rho_{bulk}$ over $\rho$ ($\rho_{bulk}$ is the bulk density of the porous substrate). For multicomponent fibers / blends and non-cylindrical fibers, a weighted average solid fiber density was used. The results were reported in gram of fluid per gram of substrate (g/g).

Calliper

The calliper of the absorbent substrates was determined by the EDANA Recommended Test Method (ERT 30.5-99) - Thickness. An Abram Model 2000 micrometer with an accuracy of 0.01 mm and lowering speed of 3 mm/s and measuring pressure of 0.1 kPa was used (Technische Beratung Abram GmbH). The same method was applied to measure the calliper of foam substrates.

Basis Weight

The basis weight of the absorbent substrates was determined by the EDANA Recommended Test Method (ERT 40.3-90) - Mass per unit area. A substrate test area of 100 x 100 mm was accurately cut and weighed. The results reported as grams per square meter (g/m$^2$). The same method was applied to measure the basis weight of foam substrates.

Table 5: Technical characteristics of some absorbent substrates.

| Absorbent substrate | Median pore radius [microns] | Absorbent capacity [g/g] | Calliper [mm] | Basis weight [g/m$^2$] |
|---|---|---|---|---|
| Libeltex 01-766 01-766 DI-08 | 550 | 41 | 6.24 | 148 |
| PGI FB-215 | 700 | 57 | 7.83 | 136 |
| PGI FB-185 | 500 | 45 | 7.17 | 156 |
| Recticel Bulpren Foam S28280 | 1400 | 40 | 6.02 | 143 |
| Polyscorer Foam 410* | 2200 | 29 | 11.12 | 365 |

(continued)

| Absorbent substrate | Median pore radius [microns] | Absorbent capacity [g/g] | Calliper [mm] | Basis weight [g/m²] |
|---|---|---|---|---|
| PGI FB-213A | 700 | 41 | 4.94 | 118 |
| BBA Fiberweb Tenotex PL60L* | 70 | 7 | 0.46 | 59 |
| Freundenberg AL 1060 | 300 | 16 | 0.99 | 60 |

Mixing of hair treatment compositions method

When two or more hair treatment compositions are mixed prior to loading into the absorbent substrate as described herein the following procedure may be used. Said at least two hair treatment compositions, which may either be both liquid or one liquid and one powder, are placed into a vessel in a weight ratio between 1:10 and 10:1. This vessel may be of different forms, for example a bottle with a lid or an open compartment. In the case of the bottle, the two compositions are shaken for 2 minutes until the mixture is homogeneous. In the case of the open compartment the two compositions are mixed with a mixing implement for a period of time sufficient to produce a homogeneous mixed product. The mixed product produced via this method is the same as that obtained when the two compositions are loaded into separate absorbent materials and mixed as the absorbents are pressed together.

Viscosity measurement test method

The viscosity of the hair treatment composition to be loaded into the absorbent substrate was measured using a Brookfield viscometer with cone and plate attachment. For viscosities in the range of about 0 cPs to 12,000 cPs the Brookfield DV-II+ viscometer with S42 plate was used. A sample of 2 ml of the hair treatment composition or Carbopol® 956 solution was equilibrated at 1 rpm at 26.7 °C for one minute prior to measurement, whereupon the readings are taken at 1 rpm. For viscosity values of about 12,000 cPs, another measurement is taken as described herein below.

For viscosities in the range of 12,000 cPs to about 100,000 cPs the Brookfield DV-II+ viscosities with S52 plate was used. A sample of 0.5 ml of the hair treatment composition or Carbopol® 956 solution was equilibrated at 1 rpm at 26.7 °C for one minutes prior to measurement, whereupon the readings are taken at 1 rpm. Again as explained above for viscosity values of 100,000 cPs, another measurement is taken as described herein below.

For viscosities in the range of 100,000-150,000 cPs the Brookfield DV-II+ viscometer with S52 plate is used. A sample of 0.5 ml of the hair treatment composition or Carbopol® 956 solution is equilibrated at about at 0.5 rpm 26.7 °C for one minutes prior to measurement, whereupon the readings are taken at 0.5 rpm.

Minimum viscosity limit test

All absorbent substrates used in the following test methods had a calliper of from about 6 mm to about 10 mm. The required calliper was achieved for BBA Fiberweb Tenotex P101 and Freudenberg AL 1060 absorbent substrate by laminating multiply plies of the absorbent substrates by means of a hot melt adhesive positioned in circular droplets not greater than about 2 mm of diameter on each surface. The lamination was performed at the perimeter of the absorbent substrates.

The absorbent substrates were cut into circular pads of about 3.57 cm of diameter (surface area 10.0 cm²), weighed and placed on the 4 cm diameter sinter glass disc of a about 80 cm³ Pyrex Buchner funnel (available from Fisher; code FPJ-400-110D). The Carbopol® 956 solution was carefully added onto the absorbent substrate to a depth of about 1 cm after which a hand bellow, attached with a single hole rubber bung fitting into the opening of the funnel, were inflated and used to force the fluid into the absorbent substrate for about 1 minute. The absorbent substrate so loaded was removed carefully from the funnel with tweezers. Excess surface fluid was removed by placing two layers of laboratory tissue paper on a bench top and carefully passing the loaded absorbent substrate (handled with tweezers) over the tissue for a distance of about 30 cm, then turning the absorbent substrate and repeating for the other side. The loaded absorbent substrate was weighed and centrally fixed by means of 3M double sided tape onto the flat side of the circular test plate of about 4.97 cm diameter (made from rigid white acetal). The opposite plate was fitted with three brass pins appropriately selected such that the absorbent was compressed by about 3 mm when the surfaces of the two plates are parallel and vertically compressed. The brass pin height was set to about 3 mm for BBA Fiberweb Tenotex P101 and Libeltex 01-766 DI-8 whilst it was of about 7 mm for Recticel Bulpren S28280. The plate containing the brass pins was secured above a weighing boat with its surface perpendicular to the lab bench and the empty fourth pin hole in the lowest position vertical to the top brass pin. The plate containing the absorbent substrate was then vertically compressed against the pin plate for about 1 minute. The weight of the weighing tray and of any dripped hair treatment composition or Carbopol® 956 solutions was recorded and the percentage of the total fluid loaded that dripped out during the compression was calculated. The measurement was repeated three times for each experiment.

Determination of the minimum viscosity limit: Consumer Panellist Study

Libeltex 01-766 DI-8 absorbent substrate was cut into two circular pads of about 3.57 cm diameter (surface area 10.0 cm$^2$) and loaded with Carbopol® 956 solutions having viscosities of about 1,000 cPs, about 2,500 cPs or about 5,000 cPs as described above. The loaded Libeltex 01-766 DI-8 absorbent substrate was centrally fixed to two hinged plates by means of 3M double sided tape. The panellists were given the application tool so prepared and asked to swipe twice a bundle of human hair of about 0.75 g in weight and of about 30.5 cm in length (Caucasion Light Brown - International Hair Imports and Products, Valhalla, New York). Its performance was evaluated with the question "Considering the sample you have just used, how would you rate its mess?" Answers were rated on a 0 to 8 scale (0 = not messy at all; 8 = extremely messy).

Minimum median pore radius limit test

The absorbent substrate samples (BBA Fiberweb Tenotex P101; Freudenberg AL 1060; Libeltex 01-766 DI-8; PGI FB-215 and Recticel Bulpren S28280), were cut into circular pads of 2.53 cm diameter (surface area of about 5.03 cm$^2$), weighed and placed on the sintered glass filter disc in a 30 cm$^3$ Duran Filter Assembly (Duran reference 24.720/24). A Viton ring was then placed upon the absorbent substrate such that the exposed absorbent had a diameter of about 1.8 cm (about 2.54 cm$^2$). The Duran Filter Assembly was attached and filled with the Carbopol® 956 solutions to a depth of about 1 cm by slow addition down the side of the glass head avoiding absorbent disturbance. Additional Carbopol® 956 solution was then poured into the glass head up to the graduated 30 cm$^3$ mark. After about 5 minutes the remaining fluid was separated and the absorbent substrates removed with tweezers. Any excess of surface fluid was removed by carefully passing the absorbent substrate over two layers of laboratory tissue paper a distance of about 30 cm for each side. The loading (grams of Carbopol® 956 solution per gram of absorbent substrate) was calculated based on the weight of the exposed absorbent substrate (surface area of about 2.54 cm$^2$) as follows and reported to two decimal places:

$$\text{Loading [g/g]} = \frac{2 \times \left(\text{AS weight after loading}\right) - \left(\text{AS weight before loading}\right)}{\left(\text{AS weight before loading}\right)}$$

wherein AS means absorbent substrate.

Maximum viscosity limit test

The Recticel Pottscorer 410 and Recticel Bulpren S28280 foams were cut into two circular pads of 3.57 cm diameter (surface area 10.0 cm$^2$) and loaded with a 100,000 cPs Carbopol® 956 solution as described in the determination of the minimum viscosity limit test method. The loaded pads were then centrally fixed, by means of 3M double sided tape, onto the flat sides of two test plates of 4.97 cm diameter. One of the plates was fitted with three 12 mm pins and secured on a clamp stand with the loaded Recticel Pottscorer 410 foam's surface perpendicular to the lab bench and the empty fourth pin hole at the lowest position. A 0.75 g bundle of hair (30.5 cm in length fanned to a width of 3 cm) was vertically compressed between the Recticel Pottscorer 410 and Recticel Bulpren S28280 foams on the pin plate and the second parallel plate. The hair was swiped twice in between the compressed foam such that the whole length of the hair bundle took three seconds to pass through. This was repeated on a different second and a third hair bundle. Each experiment as described above was repeated three times. The weights of the hair bundles were recorded and the results calculated as grams of Carbopol® 956 solution deposited per gram of hair.

9. EXAMPLES

Several exemplary hair treatment compositions are described below for incorporation within one or more exemplary absorbent substrates. The absorbent substrates of the following examples can be optionally adhered to an application tool. Preferably, the absorbent substrates are mounted onto two injection moulded polypropylene plates of about 12.5 cm$^2$ surface area, hinged together, with the absorbent substrates facing one another.

Example A: Hair highlighting using peroxide and alkalizer compositions via an absorbent substrate

Two circular disks of about 12.5 cm$^2$ are cut from an about 150 grams-per-square meter polyester high loft batting non-woven substrate (01-766 DI-8 available from Libeltex, Belgium; FB-215 available from PGI, New Jersey). Also polyurethane sponge (Bulpren S28280 available from Recticel International, Belgium) may be employed within this example.

| Peroxide composition (1) | % w/w | Alkalizer composition (2) | % w/w |
|---|---|---|---|
| De-ionized Water | q.s. to 100% | De-ionized Water | q.s. to 100% |
| | | Ammonium Hydroxide | |
| Glycerine | 5.00 | | 0.00 |
| | | (30% Active) | |
| Hydrogen Peroxide | | Ammonium bicarbonate | |
| | 17.20 | | 20.0 |
| (35% Active) | | (100% active) | |
| Disodium EDTA | 0.04 | Carbopol® 956 | 1.80 |
| Carbopol® 956 | 2.25 | pH | 8.80 |
| Sodium Hydroxide | | | |
| | q.s. to pH 2.9 | | |
| (50% aq. Solution) | | | |
| Viscosity | 15,400 cPs | Viscosity | 8,100 cPs |
| Viscosity of compositions (1) and (2) when mixed = 24,000 cPs | | | |

The Carbopol® 956 used to prepare the peroxide composition (1) is hydrated in rapidly mixing water until homogenous either by slow manual addition or by using an eductor or similar device for rapid hydration of powders. This example specifically reports Carbopol® 956 as the thickening agent for the peroxide and alkalizer compositions (1) and (2), but other thickening agents are contemplated. The hydrogen peroxide is then added with moderate mixing so as not to introduce excess air bubbles into the system. Then, 50% sodium hydroxide is added dropwise as appropriate to adjust the pH to the indicated value. Optionally, additional peroxide stabilizers such as sodium stannate may be added to further reduce the likelihood of premature peroxide decomposition. Once the peroxide composition (1) is prepared the viscosity according to the test method described herein above is measured.

The alkalizer composition (2) is produced by hydrating the Carbopol® 956 in rapidly mixing water either by slow manual addition or by using an eductor or similar device for rapid hydration of powders. When the Carbopol® 956 is fully dispersed and homogenous, the ammonium hydroxide or ammonium bicarbonate is added with moderate mixing so as to avoid entrapping excess air bubbles. The batch will thicken and clear with the addition of the alkalizer. Once the alkalizer composition (2) is prepared the viscosity according to the test method described herein above is measured.

About 4 grams of peroxide composition (1) are loaded into one of the two absorbent substrate disks and approximately 4 grams of the alkalizer composition (2) are loaded into the other absorbent substrate disk. The compositions are loaded by even application across the absorbent substrate surface with a pipette or syringe with gentle mechanical pressing with the pipette or syringe to ensure that the majority of the composition is fully absorbed into the substrate and without sacrificing the integrity of the absorbent substrate.

The plates are pressed together from two to ten times so to compress and squeeze the absorbent substrates and to sufficiently mix the peroxide composition (1) and the alkalizer composition (2).

Once the hair treatment application system is ready it is used to treat the hair according to the hair treatment test described below. The hair so treated compared to a control hair tress was visually lighter.

Hair treatment test

A tress of human hair (Caucasion Light Brown - International Hair Imports and Products, Valhalla, New York) of about 30.5 cm in length and about 0.8 gram in weight is prepared by binding one end of the hair strands with a plastic cable tie about 2 cm from the hair ends and further securing with a 3 cm strip of electrician's tape. This hair tress is hung on a stainless-steel slotted holder. The top of the hair tress is then contacted with the hair treatment application system so that the tress is between the two absorbent substrates attached to the plates. The hair treatment application system is then pulled through to the end of the hair tress while maintaining the plates juxtaposed. The application may be repeated a second time. The resulting hair tress, which has between about 0.3 to about 0.8 grams of the hair treatment compositions deposited onto it, is then placed on a weigh boat and stored in an oven at about 30°C for about 30 minutes. The hair tress is then rinsed with water and left to dry.

Example B: Hair coloured highlights using peroxide and oxidative dyes compositions via an absorbent substrate.

Absorbent substrates are prepared as described in Example A above.

| Peroxide Composition (3) | % w/w | Oxidative Dyes + Alkalizer Composition (4) | % w/w |
|---|---|---|---|
| De-ionized Water | q.s. to 100% | De-ionized Water | q.s. to 100% |
| Glycerine | 5.00 | Ethanolamine | 4.00 |
| Hydrogen Peroxide (35% Active) | 17.20 | Propylene glycol | 5.00 |
| Disodium EDTA | 0.04 | Carbopol® 956 | 1.00 |
| Carbopol® 956 | 1.00 | Glycerine | 5.00 |
| Sodium Hydroxide (50% aq. Solution) | q.s. to pH 3.5 | Sodium Sulphite | 0.30 |
| Viscosity | 10,500 cPs | EDTA | 0.10 |
| | | Erythorbic acid | 0.40 |
| | | 1-Naphthol | 0.10 |
| | | Para-aminophenol | 0.85 |
| | | 1-hydroxy-4,5-diaminopyrazole sulfate | 0.30 |
| | | Phenyl Methyl Pyrazolone | 0.20 |
| | | 2-methyl-5-hydroxyethyl aminophenol | 1.50 |
| | | pH | 10.0 |
| | | Viscosity | 6,800 cPs |
| Viscosity of compositions (3) and (4) when mixed = 17,100 cPs | | | |

The peroxide composition (3) is produced by combining the Carbopol® 956 with the glycerine and mixing until a homogenous slurry is obtained. De-ionized water is charged into a separate container of sufficient size to contain the entire batch. The slurry is introduced into the water slowly and mixed with moderate agitation until a stable, homogenous composition is observed. The hydrogen peroxide is then added with moderate mixing so as not to introduce excess air bubbles into the system. Then, sodium hydroxide is added dropwise to increase the pH to about 3.5. Optionally, additional peroxide stabilizers such as sodium stannate may be added to further reduce the likelihood of premature peroxide decomposition.

Oxidative dye and alkalizer composition (4) is produced by hydrating the Carbopol® 956 in rapidly mixing water either by slow manual addition or by using an eductor or similar device for rapid hydration of powders. When the Carbopol® 956 is fully dispersed and homogenous, all the remaining ingredients are added, apart from the ethanolamine (i.e. glycerine, dye precursors, pH buffers and antioxidants). Once they have dissolved, the ethanolamine is added with moderate mixing so as to avoid entrapping excess air bubbles. The batch will thicken and clear with the addition of the alkalizer. A hair tress is treated as described above in the hair treatment test. A light copper shade was visually determined on the treated tress when compared to a control hair tress.

Example C: Hair highlights using persulfates and peroxide composition via an absorbent substrate.

Absorbent substrates are prepared as described in Example A above.

Approximately 6 grams of peroxide composition (3) are mixed with about 4 grams of persulfate powder 5 in a weigh boat with a spatula. Approximately 4 grams of the resulting mixture are loaded into each of the absorbent substrates and a hair tress is treated as described above in the hair treatment test.

| Persulfate powder (5) | % w/w |
|---|---|
| Ammonium Persulfate | 28.6 |
| Potassium Persulfate | 50.0 |
| Sodium Persulfate | 7.1 |
| Sodium Metasilicate | 14.3 |
| Viscosity of compositions (3) and (5) when mixed = 6,000 cPs | |

Peroxide composition (3) is prepared as described above in example B. Persulfate powders are produced by the dry blending all the ingredients, in any order, in a suitable blending apparatus such as a V-blender. The composition should be combined to homogeneity. The persulfate powder so formed is then pre-mixed with the peroxide composition (3). The mixing may be performed in a bottle, bowl or tray where the ingredients are intermixed together via shaking or stirring. The resulting composition is loaded into the absorbent substrate and then applied to the hair.

This hair highlighting composition may provide a high level of decolorizing effect in a short amount of time. The resulting hair tress has experienced significant bleaching compared to a control hair tress as determined on a Minolta Spectograph.

Example D: Root-touch-up using direct dye composition or peroxide composition with oxidative dyes via an absorbent substrate

A square piece (about 25 cm$^2$) is cut from a roll of about 150 grams-per-square meter polyester high loft batt non-woven substrate (01-766 DI-8 available from Libeltex, Belgium). The absorbent piece is adhered to the center of an about 6 x 6 cm piece of 1 mm thick clear polyethylene film with 3M two-sided tape. About 7.5 grams of direct dye composition (6) is loaded into the absorbent substrate (3,000 grams of direct dye composition (6) per square meter of absorbent substrate) on a weigh scale using a pipette or a syringe. The direct dye composition (6) is applied evenly across the absorbent substrate surface with gentle mechanical pressing with the pipette or syringe to ensure that the majority of the direct dye composition (6) is fully absorbed into the substrate. The resulting loaded absorbent substrate adhered to the polyethylene film is then rubbed into the root-line of a mannequin head (available from Salons Direct (Pro-hair)) with gloved fingers touching the polyethylene side and the absorbent substrate side being rubbed into and across the hair root-line. The resulting mannequin head is left to sit for 30 minutes and then rinsed and shampooed. The root-line of the mannequin head has experienced a light brown coloration.

| Direct Dyes Composition (6) | % w/w |
|---|---|
| De-ionized Water | q.s. to q.s. to 100% |
| HC Yellow No. 2 | 0.20 |
| Disperse Black 9 | 0.05 |
| HC Red No. 3 | 0.15 |
| Disperse Violet 1 | 0.05 |
| Erythorbic Acid | 0.03 |
| Citric Acid | 0.5 |
| Ethanolamine | 2.5 |
| Carbopol® 956 | 0.83 |
| HC Orange No. 1 | 0.1 |
| pH | 10.1 |
| Viscosity | 12,100 cPs |

Root-touch-up can be achieved with oxidative dyes by employing the exact procedure above, but with pre-mixing about 4 grams of peroxide composition (3) and about 4 grams of oxidative dye and alkalizer composition (4) as described above in example B. An amount of about 7.5 grams of resulting mixture is loaded into the absorbent substrate as described above. The resulting composition is applied on a mannequin head as described above at the root-line. After removing the composition a light brown coloration has been observed.

Non-limiting additional examples of other hair treatment compositions and cosmetic compositions having viscosity as selected herein are exemplified here below.

| Example E. Shampoo composition | % w/w |
|---|---|
| De-ionized Water | q.s. to 100% |
| Sodium laureth sulfate | 12.0 |
| Cocamidopropyl betaine | 3.0 |
| Viscosity | 3,750 cPs |

| Example F. Conditioner shampoos | % w/w |
|---|---|
| De-ionized Water | q.s. to 100% |
| Ammonium Laureth Sulfate | 10.00 |
| Ammonium Lauryl Sulfate | 6.00 |
| Cocamide MEA | 0.80 |
| Cetyl Alcohol | 0.90 |
| Ethylene Glycol Distearate | 1.50 |
| Dimethicone (Viscasil 330,000) | 1.35 |
| Polyquaternium-10 (LR30M) | 0.50 |
| Polyox PEG7M | 0.10 |
| Puresyn 6 (1-decene homopolymer) | 0.30 |
| Perfume | 0.50 |
| Citric Acid | 0.04 |
| Sodium Citrate Dihydrate | 0.40 |
| Disodium EDTA | 0.10 |
| Kathon (0.5% active) | 0.01 |
| Sodium Benzoate | 0.25 |
| Sodium Chloride | q.s. to 8,000 cps |
| Ammonium Xylene Sulfonate | q.s. to 8,000 cps |
| Viscosity | 8,000 cPs |

| Example G. Conditioners | % w/w |
|---|---|
| De-ionized Water | q.s. to 100% |
| Cetyl Alcohol*1 | 1.0 |
| Stearyl Alcohol *2 | 0.6 |
| Stearamidopropyl Dimethylamine *3 | 1.0 |
| Zinc pyrithione *4 | 2.0 |
| Benzyl alcohol | 0.4 |
| Phenoxy Ethanol | 0.3 |
| Methyl Paraben | 0.2 |
| Propyl Paraben | 0.1 |
| Hydroxyethyl Cellulose | 0.3 |
| PEG-2M | 0.5 |
| Emulsifying Wax | 0.5 |
| Perfume | 0.4 |
| Citric acid | q.s. to pH 6 |
| Viscosity | 11,500 cPs |

(continued)

| Example H. Hair Styling Compositions | % w/w |
|---|---|
| De-ionized Water | q.s. to 100% |
| Acrylates/beheneth-25 Methylacrylate Copol | 2.5 |
| NATRASOL 250 | 0.6 |
| Benzyl alcohol | 0.5 |
| Acrylates Copolymer | 0.4 |
| Polyquaternium 4 | 1.4 |
| Laureth 23 (Polyoxyethylene (23) Lauryl+ | 1.6 |
| Viscosity | 57,500 cPs |

*1 Cetyl Alcohol: Konol series available from Shin Nihon Rika.
*2 Stearyl Alcohol: Konol series available from Shin Nihon Rika.
*3 Stearamidopropyl Dimethylamine: SAPDMA available from Inolex.
*4 Zinc pyrithinone: Zinc pyrithione U/2 available from Olin

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed a "40 mm" is intended to mean "about 40 mm".

## Claims

1. A hair treatment application system (10) comprising

    - at least one absorbent substrate (30) and
    - one or more hair treatment compositions (15),

    wherein said at least one absorbent substrate (30) has a median pore radius of from 300 microns to 3,000 microns and wherein said one or more hair treatment compositions (15) each has a viscosity of from 3,000 to 150,000 cps wherein said viscosity is measured before said one or more hair treatment compositions (15) are applied to said at least one absorbent substrate (30).

2. A hair treatment application system (10) according to claim 1, wherein said at least one absorbent substrate (30) has an absorption capacity of from 10 to 80 grams of liquid per gram of absorbent substrate.

3. A hair treatment application system (10) according to any one of claims 1 or 2, wherein said at least one absorbent substrate (30) has a median pore radius of from 400 microns to 2,500 microns, preferably from 450 microns to 2,000 microns, more preferably from 500 microns to 1,800 microns and wherein said one or more hair treatment compositions (15) each has a viscosity of from 5,000 cps to 125,000 cps, preferably from 7,000 cps to 100,000 cps, more preferably from 9,000 cps to 85,000 cps wherein said viscosity is measured before said one or more hair treatment compositions (15) are applied to said at least one absorbent substrate (30).

4. A hair treatment application system (10) according to claim 3, wherein said at least one absorbent substrate (30) has an absorption capacity of from 15 to 75 grams of liquid per gram of substrate, preferably from 20 to 70 even more preferably from 25 to 65 grams of liquid per gram of absorbent substrate (30).

5. A hair treatment application system (10) according to any one of the preceding claims, wherein said at least one absorbent substrate (30) is selected from non-wovens and porous foams.

**6.** A hair treatment application system (10) according to any one of the preceding claims, wherein said hair treatment application system (10) comprises a first and a second absorbent substrate (30) and wherein said first absorbent substrate is for comprising a first hair treatment composition (15) and wherein said second absorbent substrate (30) is for comprising a second hair treatment composition (15).

**7.** A hair treatment application system (10) according to any one of the preceding claims, wherein said hair treatment application system (10) further comprises an application tool (20).

**8.** A hair treatment application system (10) according to claim 7, wherein said application tool (20) comprises one plate (22), preferably two plates, more preferably two interconnected plates (22), even more preferably two plates (22) connected via a hinge (50).

**9.** A hair treatment application system (10) according to claim 8, wherein said application tool (20) comprises two plates (22), connected via a hinge (50), wherein at least one absorbent substrate (30) is attached to at least one of the two plates (22).

**10.** A method to treat the hair comprising the step of contacting the hair with said hair treatment application system (10) according to any one of claims 1 to 9.

**11.** A method according to claim 10, wherein at least one or more hair treatment compositions (15) are delivered to the hair.

**12.** A method according to any one of claims 10 or 11, wherein said at least one or more hair treatment compositions (15) are loaded into said at least one absorbent substrate (30) before contacting the hair with the hair treatment application system (10).

**13.** A method according to claim 12, wherein at least two hair treatment compositions (15) are mixed before loading of said mixed hair treatment composition (15) into said at least one absorbent substrate (30).

**14.** A method according to any one of claims 10 to 13, wherein said one or more hair treatment compositions (15) are selected from the group consisting of shampoo compositions, conditioning compositions, styling compositions, hair colourant compositions, hair bleaching compositions, highlighting compositions and combinations thereof.

**15.** A method according to any one of claims 10 to 14, wherein said hair treatment application system (10) comprises a first and a second absorbent substrate (30); wherein said first absorbent substrate (30) comprises a first hair treatment composition (15) and said second absorbent substrate (30) comprises a second hair treatment composition (15); wherein said first and second hair treatment compositions (15) are mixed by squeezing together at least once, preferably more than once, said first and second absorbent substrates (30) before application of said hair treatment composition (15) to the hair.

**16.** A method according to claims 13 or 15, wherein said first hair treatment composition (15) comprises an oxidizing agent and said second hair treatment composition (15) comprises an alkalising agent.

**17.** A kit comprising a hair treatment application system (10) according to any one of claims 1 to 9.

**18.** A kit according to claim 17, wherein said kit further comprises

    a. an individually packaged composition comprising an oxidizing agent and
    b. an individually packaged composition comprising an alkalizing agent.

**19.** A kit according to claim 18, wherein said alkalizing agent comprises a persulfate salt.

**20.** A kit according to any one of claims 17 to 19, wherein said kit further comprises at least one additional individually packaged compositions selected from the group consisting of shampoo compositions, conditioning compositions, styling compositions, hair colourant compositions, hair bleaching compositions, highlighting compositions and combinations thereof.

**21.** A kit according to any one of claims 17 to 20, wherein said kit further comprises means to mix and/or load said individually packaged compositions into said at least one absorbent substrate (30).

**22.** A kit according to any one of claims 17 to 21, wherein said kit of part further comprises instruction for the use of said hair treatment application system (10).

Fig.1.

Fig.2.

Fig.3.

Fig.4.

Fig.5.

Fig.6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20020142027 A
- US 20050079192 A
- DE 10259016
- US 6626599 B
- JP 1991178630 B

### Non-patent literature cited in the description

- International Cosmetics Ingredient Dictionary and Handbook. The Cosmetics, Toiletry, and Fragrance Association
- **SAGARIN.** Cosmetic Science and Technology. Interscience, vol. 2, 308-310
- The Encyclopaedia of Polymers and Thickeners for Cosmetics. Department of Polymer Science
- **B. MILLER ; I. TYOMKIN.** *Journal of Colloid and Interface Science,* 1994, vol. 162, 163-170
- **CHATTERJEE ; GUPTA.** Absorbent Technology. Elsevier, 2002